(19) **Europäisches Patentamt / European Patent Office / Office européen des brevets**

(11) **EP 1 392 342 B1**

(12) # EUROPEAN PATENT SPECIFICATION

(45) Date of publication and mention
of the grant of the patent:
**23.09.2015 Bulletin 2015/39**

(51) Int Cl.:
*C07K 14/47* *(2006.01)*          *G01N 33/68* *(2006.01)*
*G01N 33/92* *(2006.01)*          *C07K 1/04* *(2006.01)*
*C07K 1/107* *(2006.01)*          *C40B 30/04* *(2006.01)*

(21) Application number: **02746375.1**

(22) Date of filing: **13.05.2002**

(86) International application number:
**PCT/US2002/014982**

(87) International publication number:
**WO 2002/092118 (21.11.2002 Gazette 2002/47)**

(54) **GLOBAL ANALYSIS OF PROTEIN ACTIVITIES USING PROTEOME CHIPS**

GLOBALE ANALYSE VON PROTEINAKTIVITÄTEN UNTER VERWENDUNG VON PROTEOM-CHIPS

ANALYSE GLOBALE D'ACTIVITES PROTEIQUES AU MOYEN DE PUCES PROTEOMIQUES

(84) Designated Contracting States:
**AT BE CH CY DE DK ES FI FR GB GR IE IT LI LU MC NL PT SE TR**

(30) Priority: **11.05.2001 US 290583 P**
**26.07.2001 US 308149 P**

(43) Date of publication of application:
**03.03.2004 Bulletin 2004/10**

(73) Proprietor: **Yale University**
**New Haven, CT 06510 (US)**

(72) Inventors:
- **SNYDER, Michael**
  **Fairfield, CT 06430 (US)**
- **ZHU, Heng**
  **Baltimore, MD 021224 (US)**
- **BERTONE, Paul**
  **Hamden, CT 06514 (US)**
- **BIDLINGMAIER, Scott, M.**
  **New Haven, CT 06511 (US)**
- **BILGIN, Metin**
  **Urbana, IL 61801-3904 (US)**
- **CASAMAYOR, Antonio, J,**
  **New Haven, CT 06511 (US)**
- **GERSTEIN, Mark**
  **North Haven, CT 06473 (US)**
- **JANSEN, Ronald**
  **New Haven, CT 06511 (US)**
- **LAN, Ning**
  **Norwalk, CT 06854 (US)**

(74) Representative: **Hewson, Timothy John et al**
**Abel & Imray**
**20 Red Lion Street**
**London WC1R 4PQ (GB)**

(56) References cited:
| | |
|---|---|
| WO-A-01/83827 | US-A- 5 262 322 |
| US-A- 5 720 928 | US-A- 5 846 819 |
| US-A- 5 866 362 | US-A- 5 965 124 |
| US-A- 5 965 389 | US-A- 6 061 476 |
| US-A- 6 064 754 | US-A- 6 075 875 |
| US-A- 6 122 408 | US-A- 6 146 830 |
| US-B1- 6 197 599 | |

- ZHU HENG ET AL: "Analysis of yeast protein kinases using protein chips" NATURE GENETICS, vol. 26, no. 3, November 2000 (2000-11), pages 283-289, XP002527528 ISSN: 1061-4036
- ZHU HENG ET AL: "Protein arrays and microarrays" CURRENT OPINION IN CHEMICAL BIOLOGY, vol. 5, no. 1, February 2001 (2001-02), pages 40-45, XP002527529 ISSN: 1367-5931
- KUMAR ANUJ ET AL: "Emerging technologies in yeast genomics" NATURE REVIEWS GENETICS, vol. 2, no. 4, April 2001 (2001-04), pages 302-312, XP002527530 ISSN: 1471-0056

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ZHU H ET AL: "Global analysis of protein activities using proteome chips" SCIENCE, AMERICAN ASSOCIATION FOR THE ADVANCEMENT OF SCIENCE, US, WASHINGTON, DC, vol. 293, no. 5537, 14 September 2001 (2001-09-14), pages 2101-2105, XP002201608 ISSN: 0036-8075
- Gina Shaw: "Cheaper Chips Find a Good Fit with Hit Validation", Drug Discovery & Developement , 3 February 2005 (2005-02-03), Retrieved from the Internet: URL:http://www.dddmag.com/articles/2007/09 /cheaper-chips-find-good-fit-hit-validatio n

Remarks:

The file contains technical information submitted after the application was filed and not included in this specification

Remarks:

The file contains technical information submitted after the application was filed and not included in this specification

## Description

This invention was made with Government support under grant numbers CA77808 and GM62480 awarded by the National Institutes of Health. The Government may have certain rights in the invention.

## RELATED APPLICATIONS

This application claims benefit of United States provisional application nos. 60/290,583, filed on May 11,2001, and 60/308,149, filed on July 26,2001.

## 1. FIELD OF THE INVENTION

The present invention relates to proteome chips comprising arrays having a large proportion of all proteins expressed in a single species. The invention also relates to methods for making proteome chips. The invention also relates to methods for using proteome chips to systematically assay all protein interactions in a species in a high-throughput manner.

The present invention also relates to methods for making and purifying eukaryotic proteins in a high-density array format. The invention also relates to methods for making protein arrays by attaching double-tagged fusion proteins to a solid support. The invention also relates to a method for identifying whether a signal is positive.

## 2. BACKGROUND OF THE INVENTION

A daunting task in the post-genome sequencing era is to understand the functions, modifications, and regulation of every protein encoded by a genome (Fields et al., 1999, Proc Natl Acad Sci. 96: 8825; Goffeau et al., 1996, Science 274: 563). Currently, much effort is devoted toward studying gene, and hence protein, function by analyzing mRNA expression profiles, gene disruption phenotypes, two-hybrid interactions, and protein subcellular localization (Ross-Macdonald et al., 1999, Nature 402: 413; DeRisi et al., 1997, Science 278: 680; Winzeler et al., 1999, Science 285: 901; Uetz et al., 2000, Nature 403: 623;

Ito et al., 2000, Proc. Natl. Acad. Sci. U. S. A. 97: 1143). Although these studies are useful, transcriptional profiles do not necessarily correlate well with cellular protein levels. Thus, the analysis of biochemical activities can provide information about protein function that complements genomic analyses to provide a more complete picture of the workings of a cell (Zhu et al., 2001, Curr. Opin. Chem. Biol. 5: 40; Martzen, et al., 1999, Science 286: 1153; Zhu et al., 2000, Nat. Genet. 26 : 283; MacBeath, 2000, Science 289: 1760; Caveman, 2000, J. Cell Sci. 113: 3543).

Several groups have recently described microarray formats for the screening of protein activities (Zhu et al., 2000, Nat. Genet. 26: 283; MacBeath et al., 2000, Science 289: 1763; Arenkov et al, 2000, Anal. Biochem 278: 123). In addition, a collection of overexpression clones of yeast proteins have been prepared and screened for biochemical activities (Martzen et al., 1999, Science 286: 1153). However, thousands of individual proteins approximating an entire proteome have not been prepared, arrayed, and screened for multiple activities (Caveman, 2000, J. Cell Sci. 113: 3543).

Zhu, Heng et al. (2001) Curr. Opin. Chem. Biol. Vol 5:1; pages 40-45 provides a review of large-scale protein analysis using high throughput techniques.

Kumar, Anuj et al. (2001) Nat. Rev. Gen. vol 2:4; pages 302-312 reviews emerging technologies in yeast genomics.

WO 01/83827 discloses methods and derivatization compounds relating to the manufacture and use of high density protein chips.

Screening an entire proteome would entail the systematic probing of biochemical activities of proteins that are produced in a high throughput fashion, and analyzing the functions of hundreds or thousands of proteins samples in parallel (Zhu et al., 2000, Nat. Genet. 26: 283; MacBeath et al., 2000, Science 289 : 1763; Arenkov et al, 2000, Anal. Biochem 278: 123). Attempts to screen an entire proteome array have encountered major obstacles, including the inability to generate the necessary expression clones, and to express and purify the expressed proteins in a high-throughput fashion. In vitro assays have previously been conducted using random expression libraries or pooling strategies, both of which have shortcomings (Martzen et al., 1999, Science 286: 1153; Bussow et al., 2000, Genomics 65: 1). Specifically, random expression libraries are tedious to screen, and contain clones that are often not full-length. Another recent approach has been to generate defined arrays and screen the array using a pooling strategy (Martzen et al. 1999, Science 286: 1153). The pooling strategy obscures the actual number of proteins screened, however, and the strategy is cumbersome when large numbers of positives are identified.

Another method useful for detecting protein-protein interactions is the two-hybrid approach (Uetz et al., 2000, Nature 403: 623; Ito et al., 2000, Proc. Natl. Acad. Sci. U. S. A. 97: 1143). The types of interactions that can be detected using this approach are limited, however, because the interactions are typically detected in the nucleus.

Therefore, there remains a need in the art for the large-scale analysis of biochemical functions which would require preparing and screening, in a high-throughput manner, a comprehensive set of proteins encoded by a species's genome.

Citation or identification of any reference in Section 2, or in any other section of this application, shall not be considered an admission that such reference is available as prior art to the present invention.

## 3. SUMMARY OF THE INVENTION

The present invention provides proteome chips useful for the global study of the protein interactions of a species in a high-throughput manner. The methods and compositions of the invention are made possible by Applicants' new and unobvious discovery of a means of preparing a comprehensive set of expression constructs containing protein-coding sequences of a genome, producing the protein products in host cells in a high-throughput fashion, and analyzing the functions of a plurality of proteins in a high-throughput manner using microarrays.

The present invention is directed to proteome chips, which are positionally addressable arrays comprising a plurality of proteins, with each protein being at a different position on a solid support, wherein the plurality of proteins represents a substantial proportion of all proteins expressed in a single species, wherein translation products of one open reading frame are considered a single protein.

An advantage of using arrays, rather than performing one-by-one assays, is the ability to identify and characterize many protein-probe interactions simultaneously. Moreover, complex mixtures of probes can be contacted with a proteome chip to, for example, detect interactions in a milieu more representative of that in a cell, and to quickly evaluate many potential binding compounds.

Accordingly, the present invention provides a positionally addressable array comprising a plurality of proteins, with each protein being at a different position on a solid support, wherein the plurality of proteins comprises at least 40% of all proteins expressed in a single species and wherein the species is a Eukaryote.

In a further embodiment, the proteins are organized on the array according to a classification of proteins. The classification can be by abundance, function, functional class, enzymatic activity, homology, protein family, association with a particular metabolic or signal transduction pathway, association with a related metabolic or signal transduction pathway, or posttranslational modification.

In another embodiment, the invention provides a positionally addressable array as described above, wherein the solid support comprises glass, ceramics, nitrocellulose, amorphous silicon carbide, castable oxides, polyimides, polymethylmethacrylates, polystyrenes, or silicone elastomers.

In a further embodiment, the solid support comprises a material that helps bind the plurality of proteins to the solid support. For example, the solid support can be coated with a material that binds to an affinity tag of each protein. In a particular embodiment, the solid support comprises glutathione. In another particular embodiment, the solid support coating comprises nickel or nitrocellulose. In another particular embodiment, the solid support coating comprises glutathione and nickel. In a one embodiment, the solid support is a nickel-coated glass slide. In a preferred embodiment, the solid support is a nitrocellulosecoated glass slide. Nitrocellulose-coated glass slides for making protein (and DNA) microarrays are commercially available (e. g., from Schleicher & Schuell (Keene, NH), which sells glass slides coated with a nitrocellulose based polymer (Cat. no. 10 484 182)). In a specific embodiment, each protein is spotted onto the nitrocellulose-coated glass slide using an OMNIGRID' (GeneMachines, San Carlos, CA).

Proteins on the proteome chips preferably are fusion proteins comprising at least one affinity tag useful for purifying and/or attaching the proteins to the proteome chip.

The present invention also relates to methods for making proteome chips. Accordingly, the invention provides a method for constructing a positionally addressable array comprising the step of attaching a plurality of proteins to a surface of a solid support, with each protein being at a different position on the solid support, wherein the plurality of proteins comprises at least 40% of all proteins expressed in a single species and wherein the species is a Eukaryote.

The present invention also relates to methods for making and isolating viral, prokaryotic or eukaryotic proteins in a readily scalable format, amenable to high-throughput analysis. Preferred methods include synthesizing and purifying proteins in an array format compatible with automation technologies. Accordingly, the invention may relate to a method for making and isolating viral, prokaryotic or eukaryotic proteins comprising the steps of growing a eukaryotic cell transformed with a vector having a heterologous sequence operatively linked to a regulatory sequence, contacting the regulatory sequence with an inducer that enhances expression of a protein encoded by the heterologous sequence, lysing the cell, contacting the protein with a binding agent such that a complex between the protein and binding agent is formed, isolating the complex from cellular debris, and isolating the protein from the complex, wherein each step is conducted in a 96-well format.

The protein is preferably a fusion protein such that the heterologous sequence comprises the coding region for the protein of interest and sequences encoding a tag, such as an affinity tag. Such tags can be useful for monitoring the protein, separating the fusion protein from cellular debris and contaminating reagents, and/or attaching the protein to a proteome chip of the invention.

The present invention also relates to methods for making a positionally addressable arrays comprising the step of attaching a plurality of fusion proteins to a surface of a solid support, with each protein being at a different position on the solid support, wherein the protein comprises a first tag, a second tag, and a protein encoded by a genomic nucleic acid of an organism. The protein may be tagged with one tag at the aminoterminal end of the protein, and with a second, different tag at the carboxy-terminal end. In other embodiments, the protein is tagged with two tags at the amino-terminal end of the protein, or with two tags at the carboxy-terminal end. The protein may be tagged with one or more tags at site (s) on the protein other than the amino-or carboxyterminal end. The advantages of using double-tagged proteins include the ability to obtain highly purified proteins, as well as providing a streamlined manner of purifying proteins from cellular debris and attaching the proteins to a solid support.

Accordingly, the first tag may be a glutathione-S-transferase tag ("GST tag") and the second tag may be a poly-histidine tag ("His tag"). The GST tag and the His tag may be attached to the amino-terminal end of the protein. Alternatively, the GST tag and the His tag may be attached to the carboxy-terminal end of the protein. The GST tag and His tag can be found on either the amino-terminal or carboxy-terminal end of the protein. In certain aspects of the disclosure, the GST tag is attached to the amino-terminal end of the protein and the His tag is attached to the carboxy-terminal end.

In other aspects of the disclosure, the His tag is attached to the amino-terminal end of the protein and the GST tag is attached to the carboxy-terminal end.

Alternating the placement of an affinity tag of a fusion protein can lead to functional secondary structure, proper folding of extracellular domains, and appropriate trafficking, localization, and/or secretion of proteins. For example, fusion of a GST tag and a His tag onto the carboxy-terminal end of the protein can obviate inappropriate folding or expression when the regions upstream of the translational initiation codon are blocked.

The present invention also relates to methods of using a protein array to screen for lipid-binding proteins. Accordingly, in one aspect of the disclosure, the invention relates to a method for using a positionally addressable array comprising a plurality of proteins, with each protein being at a different position on a solid support, comprising the steps of contacting a probe with the array, and detecting protein-probe interaction, wherein the probe comprises a lipid.

In a particular aspect of the disclosure, the lipid comprises a phospholipid such as, but not limited to, phosphatidylcholine and phosphatidylinositol. In another particular aspect of the disclosure, the probe is in the form of a liposome containing phospholipids of interest.

Also disclosed are methods for using proteome chips. The proteome chips of the invention can be used to assay for essentially all protein-protein interactions in a species or cell. The proteome chips can also be used to systematically assay all the proteins of a species that interact with a test compound. Therefore, using the proteome chips of the invention, a multitude of activities can be assayed to yield a wealth of information such as, but not limited to, defining a "fingerprint" or "signature" of a cell or organism in response to a stimulus, characterizing all proteins in a species that interact with a probe of interest, characterizing all proteins in two or more species that interact with a probe of interest, characterizing all proteins involved in a biological pathway (e. g., metabolic or signal transduction pathway) or in related biological pathways, characterizing all proteins in a species with enzymatic activity (ies) of interest (e. g., kinase activity, protease activity, phosphatase activity, glycosidase, acetylase activity, and other chemical group transferring enzymatic activity), characterizing all proteins in a species with posttranslational modification (s) of interest, and identifying drug targets. In a specific aspect of the disclosure, proteome chips of the invention are used to characterize all proteins, e. g., drug targets, in a species that interact to with a drug or drug candidate of interest.

Thus, the disclosure encompasses a method for detecting a binding protein comprising the steps of contacting a probe with a positionally addressable array comprising a plurality of proteins, with each protein being at a different position on a solid support, wherein the plurality of proteins comprises at least 40% of all proteins expressed in a single species and wherein the species is a Eukaryote, and detecting any protein-probe interaction.

3.1 Definitions

As used herein, the word "protein" refers to a full-length protein, a portion of a protein, or a peptide. Proteins can be produced via fragmentation of larger proteins, or chemically synthesized. Preferably, proteins are prepared by recombinant overexpression in a species such as, but not limited to, bacteria, yeast, insect cells, and mammalian cells. Proteins to be placed in a protein microarray of the invention preferably are fusion proteins, more preferably with at least one affinity tag to aid in purification and/or immobilization.

As used herein, the word "interactor" refers to a protein on a protein microarray that interacts with a probe.

As used herein, the word "probe" refers to any chemical reagent such as, but not limited to, a protein, nucleic acid (e. g., DNA, RNA, oligonucleotide, polynucleotide), small molecule, substrate, inhibitor, drug or drug candidate, receptor, antigen, hormone, steroid, lipid, phospholipid, liposome, antibody, cofactor, cytokine, glutathione, immunoglobulin domain, carbohydrate, maltose, nickel, dihydrotrypsin, calmodulin, biotin, lectin, and heavy metal, that can be applied to a protein microarray of the invention to assay for interaction with a protein of the microarray.

## 4. BRIEF DESCRIPTION OF THE FIGURES

**FIGS**. **1A-1B.** The procedure of yeast proteome analysis using protein chip technology.

A. The yeast ORFs were cloned into a double-tagged yeast GAL1 expression vector via a recombination strategy and verified for correct identities by sequencing. Each pure plasmid construct was then reintroduced into a yeast strain for large-scale protein purification. Yeast cultures were grown in a 96-well format, and induced by adding galactose. After the high-throughput purification step, the purified proteins were aliquoted and stored in a glycerol buffer at -80°C before printing. Using a high precision microarrayer, 6566 protein samples can be spotted in duplicate onto 80 slides in a single experiment.
B. Immunoblot analysis of proteins purified from 3-ml yeast cultures.

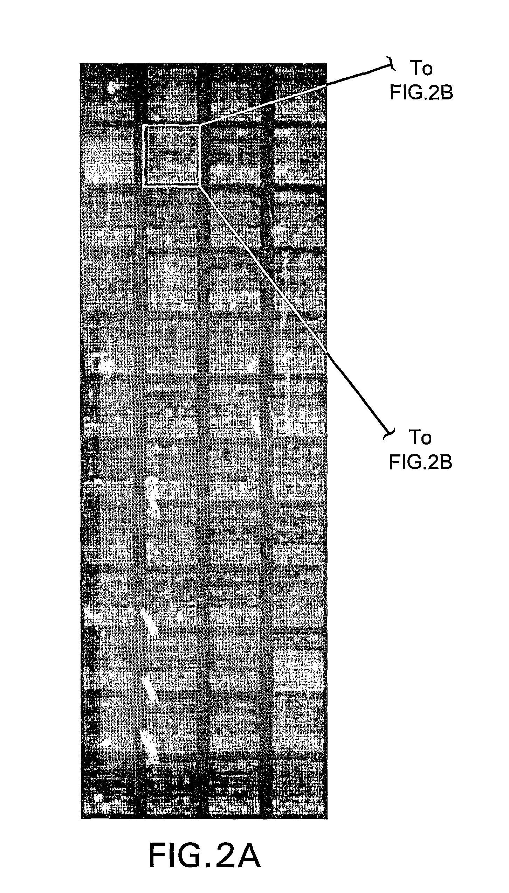

**FIGS. 2A-2C.** A proteome chip probed with anti-GST antibodies.
Sixty samples were examined by immunoblot analysis using anti-GST antibodies; 19 representative examples are shown. Greater than 80% of the preparations produce high yields of fusion protein.

A. GST::yeast fusion proteins were purified in a 96-well format. 6566 protein samples representing 5800 unique proteins were spotted in duplicate on a single nickel-coated microscope slide. The slide was probed with anti-GST antibodies.
B. An enlarged image of one of the 48 blocks is depicted to the right of the proteome chip. The letters indicate the duplicated protein samples, and the numbers represent the source plate numbers. Note the excellent resolution and high signal-to-noise ratios.
C. Distribution of errors between duplicated spots of each ORF product. The unit of $\varepsilon$ is the signal intensity determined by the Axon scanner. As determined by a GST control, ten units are equivalent to approximately 32 fg of protein. The center of the $\varepsilon$ distribution is 1.2 units, and 95% of the samples are within 10 units from the center.

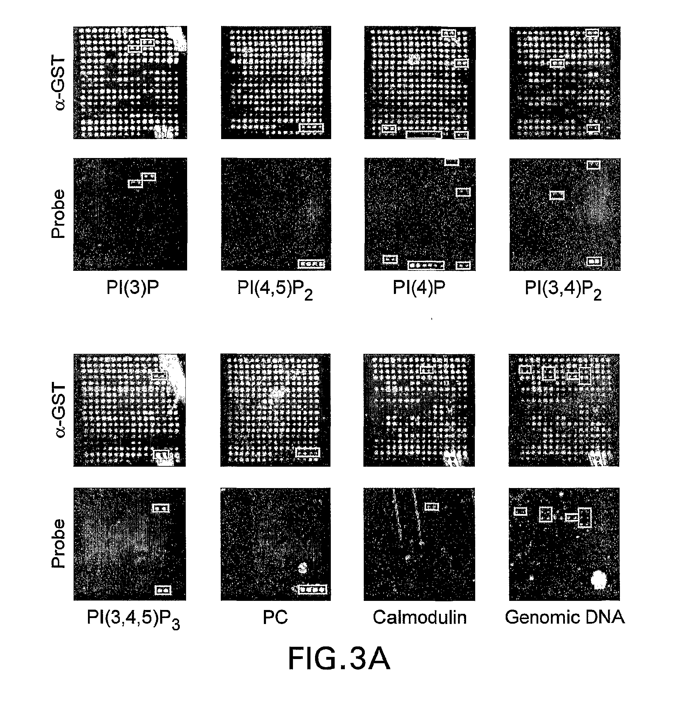

**FIGS. 3A-3B.** Examples of different assays on the proteome chips.

A. Proteome chips containing 6566 yeast proteins were spotted in duplicate and incubated with the biotinylated probes indicated. Positive interactors, indicated by boxes, were identified in six protein-liposome interaction assays ("PI(3)P", " PI(4,5)$P_2$", "PI(4)P", "PI(3,4)$P_2$", "PI(3,4,5)$P_3$", "PC"), a calmodulin-binding assay ("Calmodulin"), and a DNA-protein interaction assay ("Genomic DNA"). Each block contains 16X18 protein spots. The positive signals in duplicate appear as horizontal pairs in the bottom panels. The duplicate spotting serves as an internal control, which is important when the signals are weak relative to the background. The upper panels show the same yeast protein preparations of a control proteome chip probed with anti-GST antibodies ("$\alpha$-GST"). As demonstrated by the figure, strong signals are often observed in samples having relatively low levels of GST fusion protein ("Probe"), indicating that the binding is sensitive and specific. PI, phosphatidylinositol. PC, phosphatidylcholine.
B. A putative calmodulin-binding motif identified by searching for amino acid sequences that are shared by the different calmodulin targets (Zhu et al. 2000, Nat. Genet. 26:283). 14 of 39 positive proteins share a motif whose consensus is I/L-Q-X-K-K/X-G-B (SEQ ID NO: 1), where X is any residue and B is a basic residue. The size of the lettering, depicted above the alignment, indicates the relative frequency of the amino acid indicated. YFL003C/MSH4 (SEQ ID NO: 2); YJR073C/OPI3 (SEQ ID NO: 3); YBR050C/REG2 (SEQ ID NO: 4); YNL202W/SPS19 (SEQ ID NO: 5); YOL016C/CMK2 (SEQ ID NO: 6); BR011C/IPP1 (SEQ ID NO: 7); YGR034W/RPL26B (SEQ ID NO: 8); YFR004W/RPN11 (SEQ ID NO: 9); YIL021 W/RPB3 (SEQ ID NO: 10); YGL063W/PUS2 (SEQ ID NO: 11); YDR292C/SRP101 (SEQ ID NO: 12); YFR014C/CMK1 (SEQ ID NO: 13); YBR213W/MET8 (SEQ ID NO: 14); YAL029C/MYO4 (SEQ ID NO: 15).

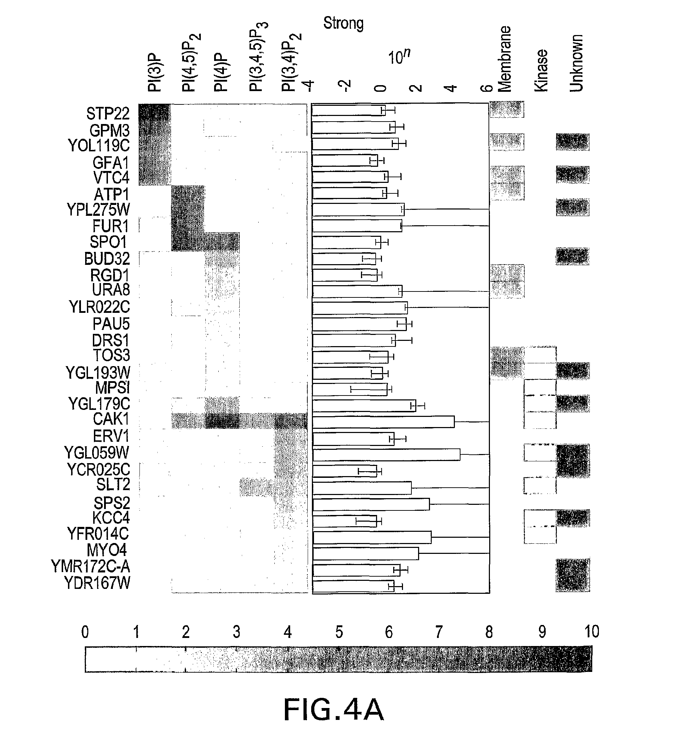

**FIGS. 4A-4D.** Analysis of the phosphatidylinositol-binding proteins. To determine the phosphatidylinositol ("PI")-binding specificity of 150 positive proteins, their binding signals were normalized against the corresponding binding signals of phosphatidylcholine ("PC"). Based on the ratios ("PI/PC"), the proteins were grouped into four categories: (A) 30 strong and specific, (B) 43 strong and nonspecific, (C) 19 weak and specific, and (D) 58 weak and nonspecific phosphatidylinositol-binding proteins. The intensity represents the PI/PC signal ratio as shown by the scale in the figure. The column, labeled "$10^n$" to the right of the PI/PC binding ratios indicates the maximum binding signal intensity (open boxes) and its confidence interval (solid horizontal lines); the numbers indicate the log of the values.

Boxes in the three columns to the right of the confidence interval column indicate membrane-associated proteins ("Membrane"), kinases ("Kinase"), and uncharacterized ORFs ("Unknown"), respectively.

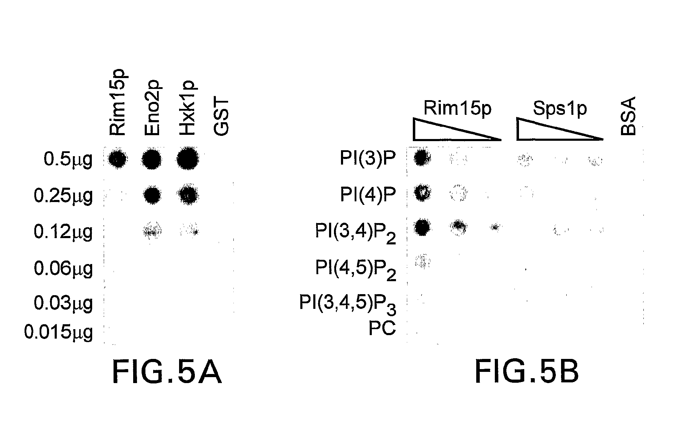

**FIGS. 5A-5C.** Conventional methods confirm protein-lipid interactions detected by the proteome microarrays (Casamayor et al., 1999, Curr. Biol. 9:186; Guerra et al., 2000, Biosci. Rep. 20:41).

A. PI(4,5)P2 liposomes were first adhered to a nitrocellulose membrane, which was blocked by BSA; a dilution series of Rim15p, Eno2p, and Hxk1p, and a GST control were used to probe the membrane. The bound proteins were detected using the anti-GST antibodies and an enhanced chemiluminescence ("ECL") kit.

B. A reverse assay was carried out to test for protein-lipid interactions. The proteins were prepared and spotted onto nitrocellulose filters in a dilution series and probed with the six different liposomes. As a control, the six liposomes were also added to the BSA-blocked membrane. After extensive washing, the bound liposomes were detected using an HRP-conjugated streptavidin and an ECL kit.

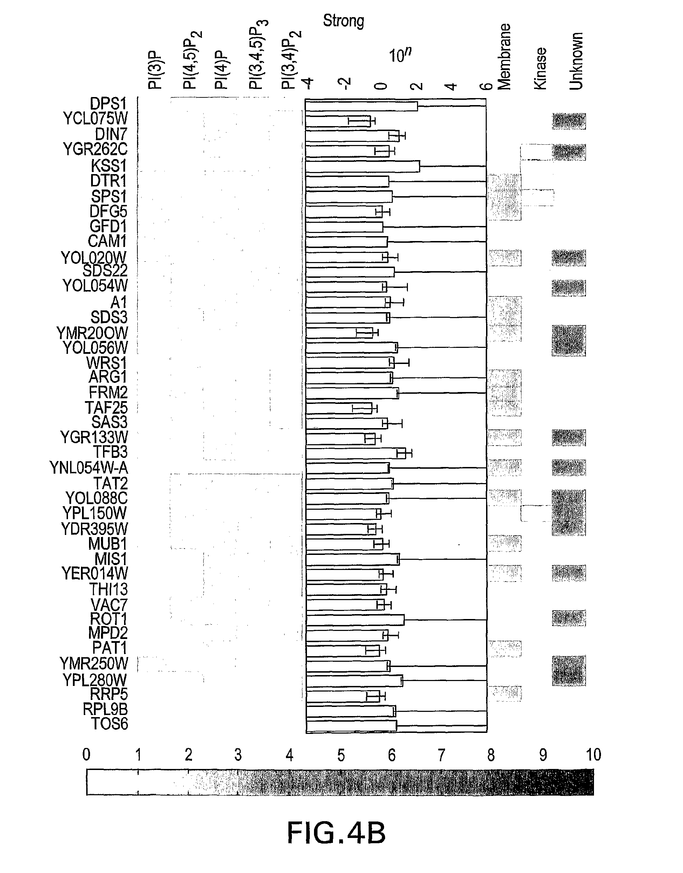

C. Linear correlation between the binding signals and the amounts of Riml 5p in a membrane assay. When liposome-binding signals of RimlSp from the membrane assay (FIG. 4B) were plotted against the concentration gradient of the spotted Riml5p, the binding signals of PI (4) P, PI (3,4) P2, PI (3) P, and PI (4,5) P2 correlated linearly with the amounts ofRiml5p. The interaction of PI (4) P and Riml 5p showed the highest affinity, which was at least three-fold higher than the affinity of the control PC with Riml5p.

## 5. DETAILED DESCRIPTION OF THE INVENTION

The present invention is based, in part, on Applicants' construction of a yeast proteome microarray containing approximately 80% of all proteins expressed in yeast, representing the first description of a proteome array for any species. The proteome chips of the invention can be used for global analyses of protein interactions and activities in a species. The use of proteome chips has significant advantages over existing approaches. One advantage of the proteome microarray technology presented here is that a large set of individual proteins can be directly screened in a high-throughput fashion for hundreds or even thousands of biochemical activities simultaneously. For example, an advantage of the proteome chip approach is that proteins can be directly screened in vitro for a wide variety of activities including, but not limited to, protein-drug interactions (e. g., drug target-drug interactions), protein-lipid interactions and enzymatic assays. In addition, a wide range of in vitro conditions can be readily tested. Furthermore, once the proteins are prepared, proteome screening is significantly faster and cheaper, and rapid data analysis in many microarray formats is compatible with existing equipment and analytical software.

### 5.1 Proteome Arrays.

The present invention encompasses a positionally addressable arrays comprising a plurality of proteins, with each protein being at a different position on a solid support, wherein the plurality of proteins comprises at least 40% of all proteins expressed in a single species, wherein the species is a Eukaryote i. e., all protein isoforms and splice variants derived from a gene are considered one protein.

A positionally addressable array provides a configuration such that each probe or protein of interest is at a known position on the solid support thereby allowing the identity of each probe or protein to be determined from its position on the array. Accordingly, each protein on an array is preferably located at a known, predetermined position on the solid support such that the identity of each protein can be determined from its position on the solid support.

In one embodiment, the species is a vertebrate. In yet another embodiment, the species is a mammal. In a particular embodiment, the species is an animal, including, but not limited to, an insect, primate, and rodent. In a specific embodiment, the species is a monkey, fruit fly, cow, horse, sheep, pig, chicken, turkey, quail, cat, dog, mouse, rat, rabbit, nematode or fish. In a preferred embodiment, the species is a human. In another preferred embodiment, the species is a yeast.

Proteins of the proteome chips of the invention include full-length proteins, portions of full-length proteins, and peptides, which can be prepared by recombinant overexpression, fragmentation of larger proteins, or chemical synthesis. Proteins can be overexpressed in cells derived from, for example, yeast, bacteria, insects, humans, or non-human mammals such as mice, rats, cats, dogs, pigs, cows and horses. Further, fusion proteins comprising a defined domain attached to a natural or synthetic protein can be used. Proteins of the proteome chips can be purified prior to being attached to the solid support of the chip. Also the proteins of the proteome chips can be purified, or further purified, during attachment to the proteome chip.

Proteins can be embedded in artificial or natural membranes (e. g., liposomes, membrane vesicles) prior to, or at the time of attachment to the protein chip. In fact, the synthesis of certain proteins may preferably be conducted in the presence of artificial or natural membranes to, for example, promote protein folding, protein processing, retain activity, and/or prevent precipitation of the protein.

Further, proteins can be attached to the solid support of the proteome chip. Alternatively, the proteins can be delivered into wells of the proteome chip, where they remain unbound to the solid support of the proteome chip.

The present invention also relates to compounds useful as solid supports for the proteome chips of the invention. The solid support can be constructed from materials such as, but not limited to, silicon, glass, quartz, polyimide, acrylic, polymethylmethacrylate (LUCITE (g)), ceramic, nitrocellulose, amorphous silicon carbide, polystyrene, and/or any other material suitable for microfabrication, microlithography, or casting. For example, the solid support can be a hydrophilic microtiter plate (e. g., MILLIPORE) or a nitrocellulosecoated glass slide. In a preferred embodiment, the solid support is a nitrocellulose-coated glass slide. Nitrocellulose-coated glass slides for making protein (and DNA) microarrays are commercially available (e. g., from Schleicher & Schuell (Keene, NH), which sells glass slides coated with a nitrocellulose based polymer (Cat. no. 10 484 182)). In a specific embodiment, each protein is spotted onto the nitrocellulose-coated glass slide using an OMNIGRID (GeneMachines, San Carlos, CA). The present invention contemplates other solid supports useful for constructing a protein chip, some of which are disclosed, for example, in co-pending United States Application No. 09/849, 781, which was filed on May 4, 2001.

In a particular embodiment, the solid support comprises a silicone elastomeric material such as, but not limited to, polydimethylsiloxane ("PDMS"). An advantage of silicone elastomeric materials is their flexible nature.

In another particular embodiment, the solid support is a silicon wafer. The silicon wafer can be patterned and etched (*see, e.g.,* G. Kovacs, 1998, Micromachined Transducers Sourcebook, Academic Press; M. Madou, 1997, Fundamentals of Microfabrication, CRC Press. The etched wafer can be used to cast the proteome chips of the invention.

In one embodiment, the present invention provides a proteome chip comprising a solid support that is a flat surface such as, but not limited to, a glass slide. Dense protein arrays can be produced on, for example, glass slides, such that assays for the presence, amount, and/or functionality of proteins can be conducted in a high-throughput manner.

Accordingly, in one embodiment, the proteome chip comprises a plurality of proteins that are applied to the surface of a solid support, wherein the density of the sites at which protein are applied is at least 100 sites/cm$^2$, 1000 sites/cm$^2$, 10,000 sites/cm$^2$, 100,000 sites/cm$^2$, 1,000,000 sites/cm$^2$, 10,000,000 sites/cm$^2$, 25,000,000 sites/cm$^2$, 10,000,000,000 sites/cm$^2$, or 10,000,000,000,000 sites/cm$^2$. Each individual protein sample is preferably applied to a separate site on the chip. The identity of the protein(s) at each site on the chip is/are known.

In another embodiment, the solid support has an array of wells. The use of microlithographic and micromachining fabrication techniques (*see, e.g.,* co-pending United States Application No. 09/849,781, filed on May 4, 2001, which is incorporated herein by reference in its entirety) can be used to create well arrays with a wide variety of dimensions ranging from hundreds of microns down to 100 nm or even smaller, with well depths of similar dimensions. In one embodiment, a silicon wafer is micromachined and acts as a master mold to cast wells of 400 $\mu$m diameter that are spaced 200 $\mu$m apart, for a well density of about 277 wells per cm$^2$, with individual well volumes of about 30 nl for 100 $\mu$m deep wells.

In another embodiment, microlithographic micromachining is used to fabricate wells 500 nm and 275 nm diameter, spaced 1 $\mu$m apart to yield well densities of over 44 million and over 61 million wells per cm$^2$ respectively. Higher densities are possible through closer spacing, as well as through smaller diameters.

In another embodiment, precision laser micromachining techniques can be used to directly fabricate mold structures out of acrylic with dimensions ranging from greater than 1.5 mm down to 500 $\mu$m, with well spacing of about 500 $\mu$m. Volumes of these wells are in the 50-500 nl range.

Accordingly, in one embodiment, the proteome chip comprises a plurality of wells on the surface of a solid support, wherein the density of wells is at least 100 wells/cm2, 1000 wells/cm', 10,000 wells/cm2, 100,000 wells/cm2, 1,000,000 wells/cm2, 10,000,000 wells/cm2, 25,000,000 wells/cm2, 10,000,000,000 wells/cm2, or 10,000,000,000,000 wells/cm2. The present invention contemplates variations of protein chips comprising a plurality of wells, which are disclosed, for example, in United States Patent No US 8399383, filed on May 4, 2001.

The present invention also contemplates variations in the shape, width-to-depth ratio and volume of wells in the proteome chip, which are disclosed, for example, in United States Patent No US 8399383, filed on May 4, 2001. Such shapes include, but are not limited to circular, oval, rectangular, square, etc. The wells can also have, for example, square, round, V-shaped or U-shaped bottoms.

In one embodiment, the solid support comprises gold. In a preferred embodiment, the solid support comprises a gold-coated slide. In another embodiment, the solid support comprises nickel. In another preferred embodiment, the solid support comprises a nickel-coated slide. Solid supports comprising nickel are advantageous for purifying and attaching fusion proteins having a poly-histidine tag ("His tag"). In another embodiment, the solid support comprises nitrocellulose. In another preferred embodiment, the solid support comprises a nitrocellulose-coated slide.

The invention further relates to compounds useful for derivatization of the proteome chip substrate. The proteins can be bound directly to the solid support, or can be attached to the solid support through a linker molecule or compound. The linker can be any molecule or compound that derivatizes the surface of the solid support to facilitate the attachment of proteins to the surface of the solid support. The linker may covalently or non-covalently bind the proteins or probes to the surface of the solid support. In addition, the linker can be an inorganic or organic molecule. In certain embodiments, the linker may be a silane, e. g., sianosilane, thiosilane, aminosilane, etc. The present invention contemplates compounds useful for derivatization of a protein chip, some of which are disclosed, for example, in United States Patent No. US 8399383, which was filed on May 4, 2001.

Accordingly, in one embodiment, the proteins of the proteome chip are bound non-covalently to the solid support (e. g., by adsorption). Proteins that are non-covalently bound to the solid support can be attached to the surface of the solid support by a variety of molecular interactions such as, for example, hydrogen bonding, van der Waals bonding, electrostatic, or metal-chelate coordinate bonding. In a particular embodiment, proteins are bound to a poly-lysine coated surface of the solid support. In addition, as described above, in certain embodiments, the proteins are bound to a silane (e.g., sianosilane, thiosilane, aminosilane, etc.) coated surface of the solid support.

In addition, crosslinking compounds commonly known in the art, e.g. homo- or heterofunctional crosslinking compounds (e.g., bis[sulfosuccinimidyl]suberate, N-[gamma-maleimidobutyryloxy]succinimide ester, or 1-ethyl-3-[3-dimethylaminopropyl]carbodiimide), may be used to attach proteins to the solid support via covalent or non-covalent interactions.

In another embodiment, the proteins of the proteome chip are bound covalently to the solid support. For example, the proteins can be bound to the solid support by receptor-ligand interactions, which include interactions between antibodies and antigens, DNA-binding proteins and DNA, enzyme and substrate, avidin (or streptavidin) and biotin (or biotinylated molecules), and interactions between lipid-binding proteins and phospholipids (or membranes, vesicles, or liposomes comprising phospholipids).

Purified proteins can be placed on an array using a variety of methods known in the art. In one embodiment, the proteins are printed onto the solid support. In a further embodiment, the proteins are attached to the solid support using an affinity tag. Use of an affinity tag different from that used to purify the proteins is preferred, since further purification is achieved when building the protein array.

Accordingly, in a preferred embodiment, proteins of the proteome chip are expressed as fusion proteins having at least one heterologous domain with an affinity for a compound that is attached to the surface of the solid support. Suitable compounds useful for binding fusion proteins onto the solid support (*i.e.,* acting as binding partners) include, but are not limited to, trypsin/anhydrotrypsin, glutathione, immunoglobulin domains, maltose, nickel, or biotin and its derivatives, which bind to bovine pancreatic trypsin inhibitor, glutathione-S-transferase, Protein A or antigen, maltose binding protein, poly-histidine (e.g., HisX6 tag), and avidin/streptavidin, respectively. For example, Protein A, Protein G and Protein A/G are proteins capable of binding to the Fc portion of mammalian immunoglobulin molecules, especially IgG. These proteins can be covalently coupled to, for example, a Sepharose® support to provide an efficient method of purifying fusion proteins having a tag comprising an Fc domain.

In a further embodiment, the proteins are bound directly to the solid support. In another further embodiment, the proteins are bound to the solid support via a linker. In a particular embodiment, the proteins are attached to the solid support via a His tag. In another particular embodiment, the proteins are attached to the solid support via a 3-glycidooxypropyltrimethoxysilane ("GPTS") linker. In a specific embodiment, the proteins are bound to the solid support via His tags, wherein the solid support comprises a flat surface. In a preferred embodiment, the proteins are bound to the solid support via His tags, wherein the solid support comprises a nickel-coated glass slide.

The proteome chips of the present invention are not limited in their physical dimensions and can have any dimensions that are useful. Preferably, the proteome chip has an array format compatible with automation technologies, thereby allowing for rapid data analysis. Thus, in one embodiment, the proteome microarray format is compatible with laboratory equipment and/or analytical software. In a preferred embodiment, the proteome chip is the size of a standard microscope slide. In another preferred embodiment, the protein chip is designed to fit into a sample chamber of a mass spectrometer.

## 5.2 Methods for Making and Purifying Proteins in a High Density Array

## Format.

The present invention also relates to methods for making and isolating viral, prokaryotic or eukaryotic proteins in a readily scalable format, amenable to high-throughput analysis. Preferred methods include synthesizing and purifying proteins in an array format compatible with automation technologies. Accordingly, in one embodiment, the invention provides a method for making and isolating eukaryotic proteins comprising the steps of growing a eukaryotic cell transformed with a vector having a heterologous sequence operatively linked to a regulatory sequence, contacting the regulatory sequence with an inducer that enhances expression of a protein encoded by the heterologous sequence, lysing the cell, contacting the protein with a binding agent such that a complex between the protein and binding agent is formed, isolating the complex from cellular debris, and isolating the protein from the complex, wherein each step is conducted in a 96-well format.

In one embodiment, the invention relates to a method for making a positionally addressable array comprising the step of attaching a plurality of proteins to a surface of a solid support, with each protein being at a different position on the solid support, wherein the plurality of proteins comprises at least 40% of all proteins expressed in a single species and wherein the species is a Eukaryote.

In one embodiment, each step in the synthesis and purification procedures is conducted in an array amenable to rapid automation. Such arrays can comprise a plurality of wells on the surface of a solid support wherein the density of wells is at least 10,20,30, 40,50,100,1000,10,000,100,000, or 1,000,000 wells/cm2, for example. Alternatively, such arrays comprise a plurality of sites on the surface of a solid support, wherein the density of sites is at least 10,20,30,40,50,100,1000,10,000,100,000, or 1,000,000 sites/cm2, for example.

In a particular embodiment, eukaryotic proteins are made and purified in a 96-array format (i. e., each site on the solid support where processing occurs is one of 96 sites), e. g., in a 96-well microtiter plate. In a preferred embodiment, the solid support does not bind proteins (e. g., a non-protein-binding microtiter plate).

In certain embodiments, proteins are synthesized by in vitro translation according to methods commonly known in the art.

Any expression construct having an inducible promoter to drive protein synthesis can be used in accordance with the methods of the invention. Preferably, the expression construct is tailored to the cell type to be used for transformation. Compatibility between expression constructs and host cells are known in the art, and use of variants thereof are also encompassed by the invention.

Any host cell that can be grown in culture can be used to synthesize the proteins of interest. Preferably, host cells are used that can overproduce a protein of interest, resulting in proper synthesis, folding, and posttranslational modification of the protein. Preferably, such protein processing forms epitopes, active sites, binding sites, etc. useful for assays to characterize molecular interactions *in vitro* that are representative of those *in vivo.*

Accordingly, a eukaryotic cell (e.g., yeast, human cells) is preferably used to synthesize eukaryotic proteins. Further, a eukaryotic cell amenable to stable transformation, and having selectable markers for identification and isolation of cells containing transformants of interest, is preferred. Alternatively, a eukaryotic host cell deficient in a gene product is transformed with an expression construct complementing the deficiency. Cells useful for expression of engineered viral, prokaryotic or eukaryotic proteins are known in the art, and variants of such cells can be appreciated by one of ordinary skill in the art.

For example, the InsectSelect system from Invitrogen (Carlsbad, CA, catalog no. K800-01), a non-lytic, single-vector insect expression system that simplifies expression of high-quality proteins and eliminates the need to generate and amplify virus stocks, can be used. A preferred vector in this system is pIB/V5-His TOPO TA vector (catalog no. K890-20). Polymerase chain reaction ("PCR") products can be cloned directly into this vector, using the protocols described by the manufacturer, and the proteins can be expressed with N-terminal histidine tags useful for purifying the expressed protein.

Another eukaryotic expression system in insect cells, the BAC-TO-BAC™ system (LIFETECH™, Rockville, MD), can also be used. Rather than using homologous recombination, the BAC-TO-BAC™ system generates recombinant baculovirus by relying on site-specific transposition in *E. coli.* Gene expression is driven by the highly active polyhedrin promoter, and therefore can represent up to 25% of the cellular protein in infected insect cells.

In a particular embodiment, yeast cultures are used to synthesize eukaryotic fusion proteins. Fresh cultures are preferably used for efficient induction of protein synthesis, especially when conducted in small volumes of media. Also, care is preferably taken to prevent overgrowth of the yeast cultures. In addition, yeast cultures of about 3 ml or less are preferable to yield sufficient protein for purification. To improve aeration of the cultures, the total volume can be divided into several smaller volumes (e.g., four 0.75 ml cultures can be prepared to produce a total volume of 3 ml).

Cells are then contacted with an inducer (e.g., galactose), and harvested. Induced cells are washed with cold (*i. e.,* 4°C to about 15°C) water to stop further growth of the cells, and then washed with cold *(i.e.,* 4°C to about 15°C) lysis buffer to remove the culture medium and to precondition the induced cells for protein purification, respectively. Before protein purification, the induced cells can be stored frozen to protect the proteins from degradation. In a specific embodiment, the induced cells are stored in a semi-dried state at -80°C to prevent or inhibit protein degradation.

Cells can be transferred from one array to another using any suitable mechanical device. For example, arrays containing growth media can be inoculated with the cells of interest using an automatic handling system *(e.g.,* automatic pipette). In a particular embodiment, 96-well arrays containing a growth medium comprising agar can be inoculated with yeast cells using a 96-pronger. Similarly, transfer of liquids (e.g., reagents) from one array to another can be accomplished using an automated liquid-handling device (e.g., Q-FILL™, Genetix, UK).

Although proteins can be harvested from cells at any point in the cell cycle, cells are preferably isolated during logarithmic phase when protein synthesis is enhanced. For example, yeast cells can be harvested between $OD_{600}$=0.3 and $OD_{600}$=1.5, preferably between $OD_{600}$=0.5 and $OD_{600}$=1.5. In a particular embodiment, proteins are harvested from the cells at a point after mid-log phase. Harvested cells can be stored frozen for future manipulation.

The harvested cells can be lysed by a variety of methods known in the art, including mechanical force, enzymatic digestion, and chemical treatment. The method of lysis should be suited to the type of host cell. For example, a lysis buffer containing fresh protease inhibitors is added to yeast cells, along with an agent that disrupts the cell wall (e.g., sand, glass beads, zirconia beads), after which the mixture is shaken violently using a shaker (e.g., vortexer, paint shaker).

In a specific embodiment, zirconia beads are contacted with the yeast cells, and the cells lysed by mechanical disruption by vortexing. In a further embodiment, lysing of the yeast cells in a high-density array format is accomplished using a paint shaker. The paint shaker has a platform that can firmly hold at least eighteen 96-well boxes in three layers, thereby allowing for high-throughput processing of the cultures. Further the paint shaker violently agitates the cultures, even before they are completely thawed, resulting in efficient disruption of the cells while minimizing protein degradation. In fact, as determined by microscopic observation, greater than 90% of the yeast cells can be lysed in under two minutes of shaking.

The resulting cellular debris can be separated from the protein and/or other molecules of interest by centrifugation. Additionally, to increase purity of the protein sample in a high-throughput fashion, the protein-enriched supernatant can be filtered, preferably using a filter on a non-protein-binding solid support. To separate the soluble fraction, which contains the proteins of interest, from the insoluble fraction, use of a filter plate is highly preferred to reduce or avoid protein degradation. Further, these steps preferably are repeated on the fraction containing the cellular debris to increase the yield of protein.

Proteins can then be purified from the protein-enriched supernatant using a variety of affinity purification methods known in the art. Affinity tags useful for affinity purification of fusion proteins by contacting the fusion protein preparation with the binding partner to the affinity tag, include, but are not limited to, calmodulin, trypsin/anhydrotrypsin, glutathione, immunoglobulin domains, maltose, nickel, or biotin and its derivatives, which bind to calmodulin-binding protein, bovine pancreatic trypsin inhibitor, glutathione-S-transferase ("GST tag"), antigen or Protein A, maltose binding protein, poly-histidine ("His tag"), and avidin/streptavidin, respectively. Other affinity tags can be, for example, myc or FLAG. Fusion proteins can be affinity purified using an appropriate binding compound (i.e., binding partner such as a glutathione bead), and isolated by, for example, capturing the complex containing bound proteins on a non-protein-binding filter. Placing one affinity tag on one end of the protein (e.g., the carboxy-terminal end), and a second affinity tag on the other end of the protein (e.g., the amino-terminal end) can aid in purifying full-length proteins.

In a particular embodiment, the fusion proteins have GST tags and are affinity purified by contacting the proteins with glutathione beads. In further embodiment, the glutathione beads, with fusion proteins attached, can be washed in a 96-well box without using a filter plate to ease handling of the samples and prevent cross contamination of the samples.

In addition, fusion proteins can be eluted from the binding compound (e.g., glutathione bead) with elution buffer to provide a desired protein concentration. In a specific embodiment, fusion proteins are eluted from the glutathione beads with 30 μl of elution buffer to provide a desired protein concentration.

For purified proteins that will eventually be spotted onto microscope slides, the glutathione beads are separated from the purified proteins. Preferably, all of the glutathione beads are removed to avoid blocking of the microarrays pins used to spot the purified proteins onto a solid support. In a preferred embodiment, the glutathione beads are separated from the purified proteins using a filter plate, preferably comprising a non-protein-binding solid support. Filtration of the eluate containing the purified proteins should result in greater than 90% recovery of the proteins.

The elution buffer preferably comprises a liquid of high viscosity such as,, for example, 15% to 50% glycerol, preferably about 40% glycerol. The glycerol solution stabilizes the proteins in solution, and prevents dehydration of the protein solution during the printing step using a microarrayer.

Purified proteins are preferably stored in a medium that stabilizes the proteins and prevents dessication of the sample. For example, purified proteins can be stored in a liquid of high viscosity such as, for example, 15% to 50% glycerol, preferably in about 40% glycerol. It is preferred to aliquot samples containing the purified proteins, so as to avoid loss of protein activity caused by freeze/thaw cycles.

The skilled artisan can appreciate that the purification protocol can be adjusted to control the level of protein purity desired. In some instances, isolation of molecules that associate with the protein of interest is desired. For example, dimers, trimers, or higher order homotypic or heterotypic complexes comprising an overproduced protein of interest can be isolated using the purification methods provided herein, or modifications thereof.

Furthermore, associated molecules can be individually isolated and identified using methods known in the art (e. g., mass spectroscopy).

**5.3 Methods for Making a Proteome Array.**

The present invention is also related to methods of making proteome chips.

Accordingly, the invention relates to methods for constructing a positionally addressable array comprising the step of attaching a plurality of proteins to a surface of a solid support, with each protein being at a different position on the solid support, wherein the plurality of proteins comprises at least 40% of all proteins expressed in a single species, wherein the species is a Eukaryote, wherein the protein is all protein isoforms and splice variants derived from a gene.

The present invention contemplates a variety of solid supports cast from a microfabricated mold, some of which are disclosed, for example, in United States Patent No. US 8399383, filed on May 4, 2001.

The present invention also relates to methods for making a positionally addressable array comprising the step of attaching a plurality of proteins to a surface of a solid support, with each protein being at a different position on the solid support, wherein the protein comprises a first tag and a second tag. The advantages of using double-tagged proteins include the ability to obtain highly purified proteins, as well as providing a streamlined manner of purifying proteins from cellular debris and attaching the proteins to a solid support. In a particular embodiment, the first tag is a glutathione-S-transferase tag ("GST tag") and the second tag is a poly-histidine tag ("His tag"). In a further embodiment, the GST tag and the His tag are attached to the amino-terminal end of the protein. Alternatively, the GST tag and the His tag are attached to the carboxy-terminal end of the protein.

In yet another embodiment, the GST tag is attached to the amino-terminal end of the protein. In a further embodiment, the His tag is attached to the carboxy-terminal end of the protein. In yet another embodiment, the His tag is attached to the amino-terminal end of the protein. In a further embodiment, the GST tag is attached to the carboxy-terminal end of the protein.

In yet another embodiment, the protein comprises a GST tag and a His tag, and neither the GST tag nor the His tag is located at the amino-terminal or carboxy-terminal end of the protein. In a specific embodiment, the GST tag and His tag are located within the coding region of the protein of interest; preferably in a region of the protein not affecting the binding domain of interest.

In one embodiment, the first tag is used to purify a fusion protein. In another embodiment, the second tag is used to attach a fusion protein to a solid support. In a specific further embodiment, the first tag is a GST tag and the second tag is a His tag.

The protein preferably is a fusion protein such that the heterologous sequence comprises the coding region for the protein of interest and sequences encoding a tag, such as an affinity tag. Such tags can be useful for monitoring the protein, separating the fusion protein from cellular debris and contaminating reagents, and/or attaching the protein to a proteome chip of the invention.

Examples of inducers include, but are not limited to, galactose, enhancer-binding proteins, and other transcription factors. In one embodiment, galactose is contacted with a regulatory sequence comprising a galactose-inducible GALI promoter.

A binding agent that can be used in accordance with the invention includes, but is not limited to, a glutathione bead, a nickel-coated solid support, and an antibody. In one embodiment, the complex comprises a fusion protein having a GST tag bound to a glutathione bead. In another embodiment, the complex comprises the a fusion protein having a His tag bound to a nickel-coated solid support. In yet another embodiment, the complex comprises the protein of interest bound to an antibody and, optionally, a secondary antibody.

## 5.4 Methods for Using a Proteome Array.

The invention is also related to methods for using proteome chips to assay the presence, amount, and/or functionality of proteins present in at least one sample. Using the proteome chips of the invention, chemical reactions and assays in a large-scale parallel analysis can be performed to characterize biological states or biological responses, and determine the presence, amount, and/or biological activity of proteins. Accordingly, the proteome chips of the invention can be used to assay for essentially all protein-protein interactions in a cell, tissue, organ, system, or organism.

Additionally, the proteome chips of the invention can be used to assay for biological responses to a particular stimulus given to a host cell (i.e., a cell used to produce the fusion protein). For example, yeast cells transformed with expression vectors encoding fusion proteins can be subjected to a stimulus, after which the fusion proteins are purified and arrayed. The arrayed proteins can then be characterized by, for example, probing with any binder. The resulting binding pattern is then compared to an identical array produced from yeast cells not subjected to the stimulus, or subjected to a different stimulus. Differences in the binding patterns can be characteristic of the biological response, and can identify specific interactors of interest with respect to the biological response.

In one embodiment, proteome chips prepared from host cells, each chip representing host cells exposed to a different stimulus, are screened with a labeled lectin (e.g., concanavalin A). The pattern of protein-probe interactions, indicating the presence of glycosylated proteins, is compared to determine the effect of each stimulus on the glycosylation state of the proteins of the proteome chip.

Biological activity that can be determined using a proteome chip of the invention includes, but is not limited to, enzymatic activity (e.g., kinase activity, protease activity, phosphatase activity, glycosidase, acetylase activity, and other chemical group transferring enzymatic activity), nucleic acid binding, hormone binding, etc. High density and small volume chemical reactions can be advantageous for the methods relating to using the proteome chips of the invention.

Further information regarding biological states or responses can be obtained using the proteome chips of the invention, wherein proteins on the chip are organized according to a classification of proteins. The classification can be by abundance, function, functional class, enzymatic activity, homology, protein family, association with a particular metabolic or signal transduction pathway, association with a related metabolic or signal transduction pathway, or posttranslational modification.

Upon contacting the proteins of a proteome chip of the invention with one or more probes, protein-probe interactions can be assayed using a variety of techniques known in the art. For example, the proteome chip can be assayed using standard enzymatic assays that produce chemiluminescence or fluorescence. Various protein modifications can be detected by, for example, photoluminescence, chemiluminescence, or fluorescence using non-protein substrates, enzymatic color development, mass spectroscopic signature markers, or amplification of oligonucleotide tags.

The probe is labeled or tagged with a marker so that its binding can be detected, directly or indirectly, by methods commonly known in the art. Any art-known marker may be used, including but not limited to tags such as epitope tags, haptens, and affinity tags, antibodies, labels, etc., providing that it is not the same as the affinity tag or reagent used to attach the protein (s) of the proteome chip to the solid substrate of the chip. For example, if biotin is used as a linker to attach proteins to a proteome chip array, then another tag not present in the protein (s) of the proteome chip, e. g., His or GST, is used to label the probe and to detect a protein-probe interaction. In certain embodiments, a photoluminescent, chemiluminescent, fluorescent, or enzymatic tag is used. In other embodiments, a mass spectroscopic signature marker is used. In yet other embodiments, an amplifiable oligonucleotide, peptide or molecular mass label is used.

In a specific embodiment, the invention relates to a method for detecting a proteinprobe interaction comprising the steps of contacting a sample of labelled probe (e. g., labelled protein) with a positionally addressable array comprising a plurality of proteins, with each protein being at a different position on a solid support; and detecting any positions on the array wherein interaction between the labelled probe and a protein on the array occurs.

Accordingly, protein-probe interactions can be detected by, for example, 1) using radioactively labelled ligand followed by autoradiography and/or phosphoimager analysis; 2) binding of hapten, which is then detected by a fluorescently labelled or enzymatically labelled antibody or high-affinity hapten ligand such as biotin or streptavidin; 3) mass spectrometry ; 4) atomic force microscopy; 5) fluorescent polarization methods; 6) infrared red labelled compounds or proteins; 7) amplifiable oligonucleotides, peptides or molecular mass labels; 8) stimulation or inhibition of the protein's enzymatic activity; 9) rolling circle amplification-detection methods (Hatch et al., 1999,"Rolling circle amplification ofDNA immobilized on solid surfaces and its application to multiplex mutation detection", Genet. Anal. 15: 35-40) ; 10) competitive PCR (Fini et al., 1999,"Development of a chemiluminescence competitive PCR for the detection and quantification of parvovirus B 19 DNA using a microplate luminometer", Clin Chem. 45: 1391-6; Kruse et al., 1999, "Detection and quantitative measurement of transforming growth factor-betal (TGF-betal) gene expression using a semi-nested competitive PCR assay", Cytokine 11: 179-85; Guenthner and Hart, 1998,"Quantitative, competitive PCR assay for HIV-1 using a microplate-based detection system", Biotechniques 24: 810-6); 11) colorimetric procedures; and 12) biological assays (e. g., for virus titers).

In a particular embodiment, protein-probe interactions are detected by direct mass spectrometry. In a further embodiment, the identity of the protein and/or probe is determined using mass spectrometry. For example, one of more probes that have bound to a protein on the proteome chip can be dissociated from the array, and identified by mass spectrometry (see, e. g., WO 98/59361). In another example, enzymatic cleavage of a protein on the proteome chip can be detected, and the cleaved protein fragments or other released compounds can be identified by mass spectrometry.

In one embodiment, each protein on the proteome chip is contacted with a probe, and the protein-probe interactions are detected and quantified. In another embodiment, each protein on the proteome chip is contacted with multiple probes, and the protein-probe interaction is detected and quantified. For example, the proteome chip can be simultaneously screened with multiple probes including, but not limited to, complex mixtures (e. g., cell extracts), intact cellular components (e. g., organelles), whole cells, and probes pooled from several sources. The protein-probe interactions are then detected and quantified. Useful information can be obtained from assays using mixtures of probes due, in part, to the positionally addressable nature of the arrays of the present invention, i. e., via the placement of proteins at known positions on the protein chip, the protein to which the probe binds ("interactor") can be characterized.

One of ordinary skill in the art can appreciate many different embodiments for assaying various cellular interactions by using probes to screen the proteome chips of the invention. For example, multiple sequential screens of a proteome chip with various probes can define all proteins involved in a particular signal transduction pathway or in a specific metabolic pathway. Moreover, these assays can be useful for diagnostic, prognostic and/or therapeutic purposes.

In accordance with the methods relating to the invention, a probe can be a cell, cell membrane, subcellular organelles, protein-containing cellular material, protein, oligonucleotide, polynucleotide, DNA, RNA, small molecule (i. e., a compound with a molecular weight of less than 500), substrate, drug or drug candidate, receptor, antigen, steroid, phospholipid, antibody, immunoglobulin domain, glutathione, maltose, nickel, dihydrotrypsin, lectin, or biotin.

Probes can be biotinylated for use in contacting a protein array so as to detect protein-probe interactions. Weakly biotinylated proteins are more likely to maintain the biological activity of interest. Thus, a gentler biotinylation procedure is preferred so as to preserve the protein's binding activity or other biological activity of interest. Accordingly, in a particular embodiment, probe proteins are biotinylated to differing degrees using a biotin-transferring compound (e. g., Sulfo-NHS-LC-LC-Biotin ; PIERCE Cat. No. 21338, USA).

In addition, the probe can be an enzyme substrate or inhibitor. For example, the probe can be a substrate or inhibitor of an enzyme such as, but not limited to, kinases, phosphatases, proteases, glycosidases, acetylases, and other group transferring enzymes. After incubation of proteins on a chip with combinations of nucleic acid or protein probes, the bound nucleic acid or protein probes can be identified, for example, by mass spectrometry (Lakey et al., 1998, "Measuring protein-protein interactions", Curr Opin Struct Biol. 8:119-23).

Accordingly, various cellular responses to interaction with the proteins on a proteome chip can be assayed by probing with whole cells. For example, a proteome chip can be contacted with lymphocytes and assayed for lymphocyte activation by various means including, but not limited to, detecting antibody synthesis, detecting or measuring incorporation of $^3$H-thymidine, labeling cell surface molecules with antibodies to identify molecules induced or suppressed by antigen recognition and activation (e.g., CD23, CD38, IgD, C3b receptor, IL-2 receptor, transferrin receptor, membrane class II MHC molecules, PCA-1 molecules, HLA-DR), and identifying expressed and/or secreted cytokines.

In another example, mitogens for a specific cell-type can be determined by incubating a cell with a proteome chip. Mitotic activity can be determined, for example, by detecting or measuring incorporation of $^3$H-thymidine by a cell. Cells can be of the same cell type (*i.e.*, a homogeneous population) or can be of different cell types.

In another example, differentiation factors for a specific cell-type can be determined by incubating a cell with a proteome chip. Differentiation of a cell can be determined, for example, by visual inspection, detection of cell-surface differentiation markers using marker-specific antibodies, or identification of secreted differentiation markers.

In another example, apoptotic factors for a specific cell-type can be determined by incubating a cell with a proteome chip. Apoptosis can be assayed, for example, by visual inspection, detection of cell-surface apoptotic markers using marker-specific antibodies, or identification of secreted markers or other cellular components released into the media.

In another example, the secretory response of a cell to a protein on a proteome chip can be assayed by incubating a cell with a proteome chip of the invention. Secreted proteins and other cellular compounds can be assayed, for example, by detecting the released compounds in the media.

In another example, the ability of a protein on a proteome chip to mediate cell aggregation can be assayed, for example, by incubating one or more cells with a proteome chip of the invention, and assaying for aggregation. Also, a protein's ability to mediate an affinity to extracellular matrix can be assayed by, for example, incubating a cell and extracellular matrix components with a proteome chip, and assaying for enhanced affinity of the cell or the extracellular matrix component with a protein on the chip. Interactors identified in such assays can have a role in, for example, cancer, cell migration, synaptogenesis, dendritic growth, process extension, or axonal elongation.

In yet another example, the effect of proteins of a proteome chip of the invention on ion transport, or other small molecule transport (*e.g.*, ATP), can be determined. For example, the probe cells can be pre-loaded with a radioactively labeled ion or other small molecule, and incubated with a proteome chip of the invention. Retention or release of the radioactive label can be measured at different time points after contacting the cells with the proteins of the proteome array. Alternatively, ion transport can be detected and characterized using electrophysiological techniques known in the art.

In yet another example, cellular uptake and/or processing of proteins on the proteome chips can be assayed by, for example, incubating a cell with a proteome chip having radioactively or fluorescently labeled proteins on the chip, and measuring the increase or decrease in signal on the proteome chip, or measuring uptake of labeled protein by the cell.

Alternatively, a proteome chip of the invention can be incubated with a cell and a labeled compound of interest, such that cellular uptake and/or processing of the compound by the cell is detected and/or measured.

Interactions of small molecules (*i.e.,* compounds smaller than MW=500) with the proteins on a proteome chip also can be assayed in a cell-free system by probing with small molecules such as, but not limited to, ATP, GTP, cAMP, phosphotyrosine, phosphoserine, and phosphothreonine. Such assays can identify all proteins in a species that interact with a small molecule of interest. Small molecules of interest can include, but are not limited to, pharmaceuticals, drug candidates, fungicides, herbicides, pesticides, carcinogens, and pollutants. Small molecules used as probes in accordance with the methods of the invention preferably are non-protein, organic compounds.

In another embodiment, essentially all receptors for a particular ligand, or class of ligands, in a species can be identified by contacting a receptor of interest with a proteome chip of the invention. Alternatively, essentially all ligands in a species that are identified by a particular receptor or receptor family of interest can be identified by contacting a receptor of interest with a proteome chip of the invention. In another embodiment, essentially all proteins in a species, capable of inhibiting or blocking formation of a particular receptor-ligand complex, can be identified by contacting a receptor and its ligand with a proteome chip of the invention, and determining whether receptor-ligand interaction is inhibited as compared with the degree of receptor-ligand interaction in the absence of the protein on the chip. Detection of receptor-ligand interaction and identification of the ligand interactors can be accomplished using methods known in the art.

In another embodiment, essentially all kinase targets in a species can be identified by, for example, contacting a kinase with a proteome chip of the invention, and in the presence of labeled phosphate, detecting phosphorylated interactors using methods known in the art. Alternatively, essentially all kinases in a species can be identified by contacting a substrate that can be phosphorylated with a proteome chip of the invention, and assaying the presence and/or level of phosphorylated substrate by, for example, using an antibody specific to a phosphorylated amino acid. In another embodiment, essentially all kinase inhibitors in a species can be identified by contacting a kinase and its substrate with a proteome chip of the invention, and determining whether phosphorylation of the substrate is reduced as compared with the level of phosphorylation in the absence of the protein on the chip.

Detection methods for kinase activity are known in the art, and include, but are not limited to, the use of radioactive labels (e.g., $^{33}$P-ATP and $^{35}$S-$\gamma$-ATP) or fluorescent antibody probes that bind to phosphoamino acids.

Similarly, assays can be conducted to identify all phosphatases, and inhibitors of a phosphatase, in a species. For example, whereas incorporation into a protein of radioactively labeled phosphorus indicates kinase activity in one assay, another assay can be used to measure the release of radioactively labeled phosphorus into the media, indicating phosphatase activity.

The proteome chips of the invention can also be used to distinguish different cell types (either morphological or functional) by, for example, contacting a proteome chip with cells or cell extracts representing different populations of cells, and comparing the patterns of protein-probe interactions on the proteome chip. This approach also can be used to characterize, for example, different stages of the cell cycle, disease states, altered physiologic states (*e.g.,* hypoxia), physiological state before or after treatment (*e.g.,* drug treatment), metabolic state, stage of differentiation, developmental stage, response to environmental stimuli (*e.g.,* light, heat), response to environmental toxins (*e.g.,* pesticides, herbicides, pollution), cell-cell interactions, cell-specific protein expression, and disease-specific protein expression.

Developmental profiles of protein-protein interactions can be used to characterize signal transduction pathways, metabolic pathways, etc. involved at every development stage and elucidate transitions between developmental stages. The wealth of information provided by such studies can be used to identify drug targets for each stage, and/or tailor treatment regimens during the course of a disease.

The proteome chips of the invention can be incubated with cell extracts to characterize a particular cell type, response to a stimulus, or physiological state. Accordingly, in exemplary embodiments, a proteome chip of the invention can be contacted with a cell extract from cells treated with a compound (*e.g.*, a drug), or from cells at a particular stage of cell differentiation (*e.g.*, pluripotent), or from cells in a particular metabolic state (*e.g.*, mitotic), and assayed for kinase, protease, glycosidase, actetylase, phosphatase, and/or other transferase activity, for example.

The pattern of protein-probe interactions on the proteome chip can thereby provide a "signature" or "fingerprint" characteristic of the biological state. For example, the results obtained from such assays, comparing for example, cells in the presence or absence of a drug, or cells at several differentiation stages, or cells in different metabolic states, can provide a signature of each condition, and can provide information regarding the physiologic changes in the cells under the different conditions.

Clearly, by screening a species's proteome using a plurality of probes (*e.g.*, known mixtures of probes, cellular extracts, subcellular organelles, cell membrane preparations, whole cells, etc.), the resulting analysis of protein-probe interactions can form the basis of identifying a "fingerprint" or "signature" of the a cell-type or physiological state of a cell, tissue, organ or system. Such information can be useful for diagnosis, prognosis, drug testing, and drug discovery, for example.

Accordingly, the proteome chips of the invention can be used to determine a drug's interactions with proteins on the chip. Alternatively, the proteome chips of the invention can be used to characterize a drug's effects on complex protein mixtures such as, for example, whole cells, cell extracts, or tissue homogenates. For example, a proteome chip can be contacted with a complex protein mixture and assayed for altered interactions of the protein mixture with the proteins on the chip when compared in presence or absence of drug.

The net effect of a drug can thereby be analyzed by screening one or more proteome chips with drug-treated cells, tissues, or extracts, which then can provide a "signature" for the drug-treated state, and when compared with the "signature" of the untreated state, can be of predictive value with respect to, for example, potency, toxicity, and side effects. Furthermore, time-dependent effects of a drug can be assayed by, for example, adding the drug to the cell, cell extract, tissue homogenate, or whole organism, and applying the drug-treated cells or extracts, prepared at various timepoints of the treatment, to a proteome chip. Such assays can be useful for diagnosis or prognosis of a disease.

In particular, the proteome chips of the invention can be useful for characterizing a mode of action of a drug, determining drug specificity, predicting drug toxicity, and for drug discovery. For example, the identity of proteins that bind to a drug, and their relative affinities, can be assayed by incubating a proteome chip with a drug or drug candidate under different assay conditions, determining drug specificity by determining where on the array the drug bound, and measuring the amount of drug bound by each different protein.

The proteome chips of the invention can be used to determine a disease state by, for example, contacting a proteome chip with diseased cells, cell extracts or tissue homogenates from diseased tissue, or body fluids from a patient suffering from a disease, and comparing the pattern of protein-probe interactions on the proteome chip with that of a healthy counterpart. Such assays can provide a "signature" for the disease state, and when compared with the "signature" of the healthy state, can be of predictive value with respect to, diagnosis or prognosis of the disease. Furthermore, stages of a disease can be characterized by, for example, assaying biological preparations on the proteome chip at various stages of the disease.

Bioassays in which a biological activity is assayed, rather than binding assays, can also be conducted out on the same proteome chip, or on an identical second chip. Thus, these types of assays using the protein chips of the invention are useful for studying drug specificity, predicting potential side effects of drugs, and classifying drugs.

Further, proteome chips of the invention are suitable for screening complex libraries of drug candidates. Specifically, the proteins on the chip can be incubated with the library of drug candidates, and then the bound components can be identified, e.g., by mass spectrometry, which allows for the simultaneous identification of all library components that bind preferentially to specific subsets of proteins, or bind to several of the proteins on the chip. Additionally, the relative affinity of the drug candidates for the different proteins in the array can be determined.

Moreover, the protein chips of the present invention can be probed in the presence of potential inhibitors, catalysts, modulators, or enhancers of an observed interaction, enzymatic activity, or biological response. Using a proteome chip of the present invention, such strategic screens can identify proteins expressed in a species that, for example, block the binding of a drug, inhibit of viral infection, exhibit bacteriostatic activity, exhibit anti-fungal activity, ameliorate parasitic infection, or physiological effectors to specific categories of proteins.

Enzymatic reactions can be performed and enzymatic activity measured using the proteome chips of the present invention. In a specific embodiment, compounds that modulate the enzymatic activity of a protein or proteins on a chip can be identified. For example, changes in the level of enzymatic activity can be detected and quantified by incubating a compound or mixture of compounds with an enzymatic reaction mixture, thereby producing a signal (*e.g.*, from substrate that becomes fluorescent upon enzymatic activity). Differences between the presence and absence of a test compound can be characterized. Furthermore, the differences in a compound's effect on enzymatic activities can be detected by comparing their relative effect on samples within the proteome chip and between chips.

A variety of strategies of using the proteome chips of the present invention can be employed to determine various physical and functional characteristics of proteins. For example, the protein chips can be used to assess the presence and amount of protein present by probing with an antibody. The protein can be detected using standard detection assays such as luminescence, chemiluminescence, fluorescence, chemifluorescence, or mass spectrometry. For example, a primary antibody to the protein of interest is recognized by a fluorescently labeled secondary antibody, which is then measured with an instrument (*e.g.*, a Molecular Dynamics scanner) that excites the fluorescent product with a light source and detects the subsequent fluorescence. For greater sensitivity, a primary antibody to the protein of interest is recognized by a secondary antibody that is conjugated to an enzyme such as alkaline phosphatase or horseradish peroxidase. In the presence of a luminescent substrate (for chemiluminescence) or a fluorogenic substrate (for chemifluorescence), enzymatic cleavage yields a highly luminescent or fluorescent product which can be detected and quantified by using, for example, a Molecular Dynamics scanner. Alternatively, the signal of a fluorescently labeled secondary antibody can be amplified using an alkaline phosphatase-conjugated or horseradish peroxidase-conjugated tertiary antibody.

In one embodiment, a proteome chip of the invention can probed with antibodies directed against known proteins in one species, such that homologous proteins having recognized epitopes can be identified in the proteome of another species. Same species homologs of the interactors can be obtained by, for example, using the DNA sequence information of the homologous protein to identify the homolog in the other species. In specific embodiments, the antibody is directed against cyclin, kinase, GST, Clb5, Cla4, Ste20, Cdc42, PI(3,4)P2, PI(4)P, SPA2, CLB1, CLB2, or Cdc11.

In another embodiment, a proteome chip of the invention containing proteins of a first species can probed with a protein from a second species to identify interactors. Homologs in the second species of the interactors from the first species can then be identified and characterized by, for example, nucleotide sequence homology. Thus, where the proteome is not available for a particular species, this strategy can be used to find same species proteins that interact with a protein of interest.

The proteome chips of the invention can be used to identify essentially all substrates in a species for each enzyme found in a species. Accordingly, identifying substrates of protein kinases, phosphatases, proteases, glycosidases, acetylases, or other group transferring enzymes can be conducted on the protein chips of the present invention.

In one embodiment, protease activity can be detected by identifying, using standard assays (e.g., mass spectrometry, fluorescently labeled antibodies to peptide fragments, or loss of fluorescence signal from a fluorescently tagged substrate), peptide fragments that are produced by protease activity and released into the media. Thus, activity of group-transferring enzymes can be assayed readily by several approaches using any means of detection, which would be appreciated by one of ordinary skill in the art.

The proteome chips of the invention can be used to identify essentially all binding proteins in a species for any compound. Accordingly, proteome chips can be used to identify and characterize essentially all proteins that bind, for example, kinases, proteases, hormones, DNA, RNA, phosphatases, proteases, glycosidases, acetylases, or other group transferring enzymes. Thus, the chip can be probed with a probe, and assayed for protein-probe interaction and/or assayed for the desired activity. For example, if RNA binding is the activity of interest, the proteome chip is probed with RNA, and protein-RNA complexes are identified.

For example, the proteome chips of the invention can be used to identify essentially all proteins in a species that bind to membrane-associated proteins or other membrane-associated compounds by contacting the chip with probes such as, for example, whole cells, preparations of plasma membranes, membrane vesicles, or liposome comprising membrane components of interest, and detecting protein-probe interaction. In a particular embodiment, the probe is in the form of a liposome comprising one or more phospholipids of interest. The protein-probe interaction can be detected using techniques known in the art. The identity of the interactor and/or probe can be determined using techniques known in the art. Moreover, biological activities (e.g., enzyme activity, cell activation) can also be detected using techniques known in the art.

The identity of target proteins from pathogens (*e.g.*, an infectious disease agent such as a virus, bacterium, fungus, or parasite) or target proteins from abnormal cells (*e.g.,* neoplastic cells, diseased cells, or damaged cells) that serve as antigens in the immune response of recovering or non-recovering patients can be determined by using a proteome chip of the invention. For example, lymphocytes isolated from a patient can be used to screen chips comprising all of a pathogen's proteins. In general, these screens comprise contacting a proteome chip with a plurality of lymphocytes, wherein the proteins on the proteome chip comprise a plurality of potential antigens, and detecting positions on the chip where lymphocyte activation occurs. In a specific embodiment, lymphocytes are contacted with a pathogen's proteins on the proteome chip, after which activation of B-cells or T-cells by an antigen or a mixture of antigens is assayed, thereby identifying target antigens derived from a pathogen.

Alternatively, the proteome chips of the invention can be used to characterize an immune response by, for example, screening a proteome of an infectious organism with a patient's lymphocytes to identify the targets of a patient's B-cells and/or T-cells. For example, B-cells can be incubated with a proteome chip of the invention to identify antigenic targets for humoral-based immunity.

In another embodiment, the proteome chips of the invention can be used to detect and characterize substrates of autoimmunity or allergy-causing proteins. For example, a proteome of human proteins can be screened, with a patient's lymphocytes or with a patient's circulating antibodies, to identify the targets of a patient's B-cells and/or T-cells. Such screens can characterize autoimmunity or allergic reactions, and identify potential target drug candidates.

In one embodiment, the proteome chips of the invention are used to identify substances that are able to activate B-cells or T-cells. For example, lymphocytes are contacted with the proteome chip, and lymphocyte activation is assayed, thereby identifying substances that have a general ability to activate B-cells or T-cells or subpopulations of lymphocytes (e.g., cytotoxic T-cells).

Induction of B-cell activation by antigen recognition can be assayed by various means including, but not limited to, detecting or measuring antibody synthesis, incorporation of $^3$H-thymidine, binding of labeled antibodies to newly expressed or suppressed cell surface molecules, and secretion of factors indicative of B-cell activation (*e.g.,* cytokines). Similarly, T-cell activation in a screen using a protein chip of the invention can be determined by various assays. For example, a chromium ($^{51}$Cr) release assay can detect recognition of antigen and subsequent activation of cytotoxic T-cells (*see, e.g.*, Palladino et al., 1987, Cancer Res. 47:5074-9; Blachere et al., 1993, J. Immunotherapy 14:352-6).

The specificity of an antibody preparation can be determined through the use of a proteome chip of the invention, comprising contacting the chip with an antibody preparation, and detecting positions on the solid support where binding by an antibody in the antibody preparation occurs. The antibody preparation can be, but is not limited to, Fab fragments, antiserum, and polyclonal, monoclonal, chimeric, single chain, humanized, or synthetic antibodies. In one example, a proteome chip is probed with a monoclonal antibody to characterize its binding strength and/or its specificity. In specific embodiments, the antibody is directed against cyclin, kinase, GST, Clb5, Cla4, Ste20, Cdc42, PI(3,4)P2, PI(4)P, SPA2, CLB1, CLB2, or Cdc11.

The proteome chips of the invention are useful for identifying proteins that bind to specific molecules of biologic interest including, but not limited to, receptors for potential ligand molecules, virus receptors, and ligands for orphan receptors.

The proteome chips are also useful for detecting DNA binding or RNA binding to proteins on the chips, and for evaluating the binding specificity and strength. The DNA can be single-stranded or double-stranded. The RNA can be mRNA, hnRNA, polyA[+] RNA, or total RNA.

The proteome chips of the invention are useful for identifying proteins that are modified posttranslationally. Posttranslational modifications that can be detected using the methods of the invention include, but are not limited to, methylation, acetylation, farnesylation, biotinylation, stearoylation, formylation, lipoic acid, myristoylation, palmitoylation, geranylgeranylation, pegylation, phosphorylation, sulphation, glycosylation, sugar modification, lipidation, lipid modification, ubiquitination, sumolation, disulphide bonding, cysteinylation, oxidation, glutathionylation, pyroglutamic acid, carboxylation, and deamidation. In addition, a protein can be modified posttranslationally with pentoses, hexosamines, N-acetylhexosamines, deoxyhexoses, hexoses, and sialic acid.

In one embodiment, a proteome chip of the invention can be probed with phosphotyrosine, phosphoserine, or phosphothreonine to identify phosphorylated interactors. In another example, a proteome chip of the invention can be probed with a lectin *(e.g.,* wheat germ agglutinin) to identify glycosylated interactors *(e.g.,* N-acetylglucosamine). The phosphorylated or glycosylated interactors can be detected using methods known in the art.

The proteome chips of the invention are also useful for identifying and characterizing protein isoforms that exhibit differences in function, ligand binding, or enzymatic activity. In a particular embodiment, a proteome chip of the invention is used to characterize different binding affinities by protein isoforms derived from different alleles by assaying their activities relative to one another.

In one embodiment, consensus sequences can be determined using the proteome chips of the invention. For example, upon screening a proteome chip with a binder (which can be from a species different from that of the proteins on the chip), the amino acid sequences of the interactors can be aligned to construct a consensus sequence for the interacting domain(s). Such information can be useful, for example, for designing inhibitors of the binder-interactor interaction, or for designing novel and/or improved binders for a particular interactor or class of interactors.

In a specific embodiment, a consensus sequence for calmodulin-binding proteins is determined by screening a proteome chip with calmodulin. In a further embodiment, the consensus sequence for a calmodulin-binding protein consists of the amino acid sequence I/L-Q-X-K-K/X-G-B (SEQ ID NO: 1). In another embodiment, the consensus sequence for a calmodulin-binding protein comprises the amino acid sequence I/L-Q-X-K-K/X-G-B (SEQ ID NO: 1). In a further embodiment, the consensus sequence for a calmodulin-binding protein comprises the amino acid sequence I/L-Q-X-K-K/X-G-B (SEQ ID NO: 1), and is less than 10, 15, 30, 50, or 100 amino acid residues in length.

The proteome chips of the invention are also useful for identifying a set of potential antibacterial, antifungal, antiparasitic or antiviral compounds. For example, cell lysates or other preparations of bacteria, fungi, parasites or viruses can be contacted with a proteome array, and protein-probe interactions detected and identified. Additionally, comparing interaction patterns obtained from infectious organisms at infectious stages and non-infectious stages can identify interactions involved in infection of the host.

Screening of phage display libraries can be performed by incubating a library with the proteome chips of the present invention. The detection of clones binding to a protein on the chip can be conducted by various methods known in the art (e.g., mass spectrometry), thereby identifying clones of interest, after which the DNA encoding the clones of interest can be identified by standard methods (*see, e.g.,* Ames et al., 1995, J. Immunol. Methods 184:177-86; Kettleborough et al., 1994, Eur. J. Immunol. 24:952-8; Persic et al., 1997, Gene 187:9-18). In this manner, the chips are useful to select for cells having surface components that bind to specific proteins on the chip.

### 5.5 Kits.

The invention also relates to kits for carrying out the assay regimens of the invention.

In one embodiment, kits relating to the invention comprise one or more proteome chips of the invention. Such kits may further comprise, in one or more containers, reagents useful for assaying biological activity of a protein or molecule, reagents useful for assaying protein-probe interaction, and/or one or more probes, proteins or other molecules. The reagents useful for assaying biological activity of a protein or other molecule, or assaying interactions between a probe and a protein or other molecule, can be applied with the probe, attached to a proteome chip, or contained in one or more wells on a proteome chip. Such reagents can be in solution or in solid form. The reagents may include either or both the proteins or other molecules and the probes required to perform the assay of interest.

The proteins of the proteome chip can be attached to the surface of a flat solid support, contained in wells on a solid support, or attached to the surface of wells on the solid support. In one embodiment, the proteome chip in the kit has the proteins and/or probes already attached to the solid support. In another embodiment, the proteome chip in the kit can have the reagent (s) or reaction mixture useful for assaying biological activity of a protein or other molecule, or useful for assaying the interaction of a probe and a protein or other molecule, already attached to wells on the solid support. In yet another embodiment, the reagent (s) is not attached to the wells of the solid support, but is contained in the wells. In yet another embodiment, the reagent (s) is not attached to the wells of the solid support, but is contained in one or more containers, and can be added to the wells of the solid support. In yet another embodiment, the kit further comprises one or more containers holding a solution reaction mixture for assaying biological activity of a protein or molecule. In yet another embodiment, the kit provides a substrate (e. g., beads) to which probes, proteins or molecules of interest, and/or other reagents useful for carrying out one or more assays, can be attached, after which the substrate with attached probes, proteins, or other reagents can be placed into the wells of the chip.

### 5.6 Design of a Positive Identification Algorithm.

The present invention also relates to a method for identifying whether a signal is positive. Signals can be in any measurable form including, but not limited to, visible light, ultraviolet radiation, infrared radiation, X-rays, fluorescence, and colorimetric visualization.

In a preferred embodiment, signals are detected by mass spectrometry.

In addition, signals can be in any arrangement, and in any physical format such as, but not limited to, arrays, blots, gels, and screens. Preferably, signals are spots in a static arrangement.

In another preferred embodiment, signals are produced by fluorescence and are arranged in a grid pattern on an array. As such, a signal can be assigned a positional coordinate with respect to row and column. The rows and columns can be of any width.

The first step in filtering signals is to calculate the local foreground and background signals for each spot. The local foreground signal is emitted from the actual spot, whereas the local background signal is emitted from the area immediately bordering to the spot. The net signal, which is the local foreground signal minus the local background signal, is used in all further calculations. The local foreground and background signals can be identified by software such as GENEPIX™.

However, variations between chips, which can represent, for example, different lipid-binding experiments, and local variations on the chip (due to unequal diffusion of substrates, for example) can result in further fluctuation of the net signal intensity, resulting in different net signal distributions for different chips. To correct the variation between chips, the net signals from different chips need to be scaled into a common range. One of the several chips is chosen as a reference and the goal is to scale the net signal distributions of each chip to the range and shape of the net signal distribution of the reference chip.

For example, the lower quartile, median, and upper quartile values of the net signal distribution of each chip can be computed. Then, for each chip, the median net signal is subtracted from the net signal of each spot. Furthermore a scaling factor is computed for each chip, which is equal to the ratio of the difference between the upper and lower quartile of the specific chip and the difference between the upper and lower quartile of the reference chip. This implies that the scaling factor for the reference chip is equal to one. Then the net signals on each chip are multiplied by the chip-specific scaling factor to calculate the scaled net signals.

To correct for local variations on an array, a "neighborhood subtraction" for each spot can be performed. For example, the neighborhood region can be defined as a region of two rows above and below, as well as two columns to the left and right of a signal spot. The median signal of this region is then subtracted from the spot signal to calculate an excess signal relative to the neighborhood of the spot. Preferably, the number of spots of high signal strength in any neighborhood region is sufficient low, such that the median value is not significantly affected and is a good representation of the background signal in the neighborhood region.

Applying the neighborhood subtraction to the scaled net signals yields the scaled excess signals. In the next step, parallel samples are compared with respect to their scaled excess signals. If the difference between the average of the scaled excess signal of the two parallel samples and the scaled excess signal of one of the parallel samples is greater than three times the standard deviation of the error of the scaled excess signal, the spots belonging to the two parallel samples are excluded from further analysis. The remaining spots and their scaled excess signal then represent the set of filtered signals.

The distribution of the error of the scaled excess signals and its standard deviation can be computed as follows. A linear regression is performed on the complete set of parallel samples to determine the general linear relationship between parallel samples. Then an error value can be calculated for each parallel sample:

$$\varepsilon_G = |G2 - Gm|,$$

where

G1 and G2 represent the two scaled excess signals of the two parallel samples, Gm = (G1 + G2)/2,

and function f(G1) = a*G2 + b is the general linear relationship between the two parallel samples G1 and G2, with the parameters a and b determined by linear regression.

The complete set of error values is the set of error values for all parallel samples and represents the distribution of error values for the scaled excess signal. Then the standard deviation of this distribution can be calculated.

Finally, a pair of parallel samples is called positive if the average of their filtered signals is three standard deviations greater than the error of the scaled excess values. (Note that this threshold is independent of the threshold to determine whether parallel samples should be excluded from the set of filtered signals.)

After this filtering procedure, the filtered signal ($G$) is normalized with the GST signal ($R$), yielding the ratio $r = G/R$ which can be a measure of the binding per amount of protein and can allow for comparison of binding signals between different proteins. The specific binding ratio $r$ is sensitive to errors $\varepsilon_G$ and $\varepsilon_R$ in both the $G$ and $R$ signals. Using a Monte-Carlo procedure, 90% and 95% confidence intervals for this ratio can be calculated. The error $e_G$ of the $r$ value is related to the errors $\varepsilon_G$ and $\varepsilon_R$:

$$r + \varepsilon_R = (G + \varepsilon_G)/(R + \varepsilon_R)$$

where $\varepsilon_R$ represents the error of the ratio r.

For the Monte Carlo procedure, both the distributions of $\varepsilon_G$ and $\varepsilon_R$ must be known. The distribution of $e_G$ can be computed as explained above. The distribution of $e_R$ can be computed in the same procedure as for $e_G$ by using the GST signal pairs of parallel samples as input.

The Monte Carlo procedure is as follows:

In order to determine confidence intervals for $r + \varepsilon_R$, a population of random samples of $r + e_R$ needs to be computed. These can be derived from random samples of $\varepsilon_G$ and $\varepsilon_R$.

Random samples of $\varepsilon_G$ can be computed as follows. Samples of uniformly distributed random numbers between 0 and 1 are calculated with a standard random number generator.

From the distributions of $\varepsilon_G$ the inverse cumulative distribution function of $e_G$ is determined by standard procedures. Using the samples of random numbers as arguments for this function produces a set of random samples for $\varepsilon_G$. Likewise a set of random samples for $\varepsilon_G$ can be calculated. These random samples of $\varepsilon_G$ and $\varepsilon_R$ can be combined in the formula $r + \varepsilon_R = (G + \varepsilon_G)/(R + \varepsilon_R)$ to produce a population of random samples for $r + \varepsilon_R$.

In one embodiment, the invention provides a method for identifying whether a signal is positive, comprising the steps of determining foreground and background signals for each spot locally and determining net signals from their difference, determining the lower quartile, median, and upper quartile values of a first and second net signal distribution, subtracting the first median value from the first net signal distribution, and subtracting the second median value from the second net signal distribution to obtain a first and second subtracted value, respectively, dividing said first subtracted value by the difference between said upper and lower quartile values of said first signal distribution, and dividing said second scaled value by the difference between said upper and lower quartile values to obtain a first and second scaled value, respectively, computing a local median value of a scaled signal distribution of a neighborhood region, wherein said neighborhood region comprises a plurality of sites in the area; subtracting the local median value from the scaled signal to obtain an scaled excess value; and parallel samples of scaled excess values are excluded if the difference between one of the sample values and their average is greater than three standard deviations of the error of the scaled excess value.

The filtered values can be used to identify whether a signal is positive. Parallel samples are called "positive" if the average of the filtered values of the parallel sample is three times greater than the standard deviation of the error of scaled excess values. Filtered positive signals can then be normalized using the formula:

$$r = G/R$$

wherein G is the filtered value, *R* is a GST signal, and r represents a signal per amount of protein that can be compared among different spots. The ratio r is sensitive to the errors in G and R. This sensitivity can be assessed by calculating confidence intervals of

$$r + \varepsilon_r = G + \varepsilon_G / R + \varepsilon_R$$

$\varepsilon_G$ is the error of *G*, $\varepsilon_R$ is the error of *R,* and $\varepsilon_r$ is the error of *r*.

In a specific embodiment, a positive signal indicates protein-probe interaction. In another specific embodiment, the neighborhood region is two rows above, two rows below, two columns to the left, and two columns to the right of the signal. In another specific embodiment, data points from two parallel samples are excluded from further analysis, wherein the difference between the scaled excess signals of said samples and their average is greater than three standard deviations of the error of the scaled excess signal. Data points are also excluded if they are obviously artifactual.

## 6. EXAMPLES

A defined collection of over 5800 proteins from the budding yeast was prepared using high-throughput techniques and screened for many activities including protein-protein, protein-DNA, protein-RNA, and protein-liposome interactions. A large number of novel activities were identified, providing new information concerning known and previously uncharacterized genes.

To facilitate studies of the yeast proteome, 5800 open reading frames were cloned and overexpressed, and their corresponding proteins purified. The proteins were printed onto slides at high spatial density to form a yeast proteome microarray and screened for their ability to interact with proteins and phospholipids. Many novel calmodulin and phospholipid-interacting proteins were identified; a common potential binding motif was identified for many of the calmodulin-binding proteins. These studies demonstrate that microarrays of an entire eukaryotic proteome can be prepared and screened for large numbers of biochemical activities resulting in the identification of many novel protein functions/interactions. These microarrays can also be screened to detect protein posttranslational modifications.

### 6.1 Yeast Culture Preparation

1. Yeast glycerol stocks stored in 96-well plates at -80°C were inoculated onto a URA-agar plate (Omni, USA) using a 96-pronger.
2. The culture was allowed to grow on agar at 30°C for 48 hours, and visible colonies (2 mm diameter) can be observed.
3. A 96-pronger was used to inoculate yeast cells from agar plates to a 96-well 2 ml box in which every well contained 300 $\mu$l URA-/raffinose liquid media and a 2 mm diameter glass ball, which facilitates the uniform growth.
4. After the culture reached O.D.$_{600}$ 4.0 in about 16 hours at 30°C with vigorous shaking (300 rpm), 15 $\mu$l of the same strain was inoculated into 750 $\mu$l of URA-/raffinose liquid media in four different boxes to obtain 3 ml of culture. Again, each well contained the same glass ball to achieve aeration and even growth. The cells were grown at 30°C with vigorous shaking.
5. After 12-16 hours of growth, the culture should reach O.D.$_{600}$ 0.6 to 0.8. Cultures were discarded if the OD is over 1.0. Using an automated liquid-handling device (Q-Fill, Genetix, UK), 40% galactose stock was added to each well to a final concentration of 2% to induce the cells. The cultures were induced at 30°C for 4 hours with shaking.
6. The cells were harvested by spinning at 3000 rpm for 2-10 min, and the cell pellets were resuspended in 100-1000 $\mu$l of cold water by vortexing. Cells of the same strain were then merged from 4 wells into one. Cells were collected by spinning and resuspended in 100-1500 $\mu$l of cold lysis buffer without the protease inhibitors on ice. The washed cells were collected by a brief centrifugation, and the lysis buffer was discarded. The washed semi-dry culture was immediately stored in -80 °C freezer. The culture can be kept for weeks.

### 6.2 Protein Purification in a 96-well Fashion

1. The frozen culture in a 96-well box was transferred from -80°C to ice and 100-300 $\mu$l of zirconia beads (0.5 mm diameter from BSP, Germany) was added to each well. While the culture was still frozen, 100-500 $\mu$l of lysis buffer containing fresh protease inhibitors was added. A cap mat was used to seal each well. After thawing the culture for 5-25 min on ice, the cells in the 96-well box were vortexed 20-60 seconds for 3-6 times with 1-5 min intervals on ice. To efficiently disrupt the yeast cell wall, and to process many plates at once, a paint shaker (HARBIL™ 5G HD, 36 kg capacity, adjustable pressure and shaking time, fixed speed at 200 times per minute) was used to violently agitate the samples.
2. After spinning at 3000 rpm for 2-10 min, the supernatant was collected using wide-open tips (Fisher, USA) and transferred into a 96-well filter plate (Whatman, USA; Whatman UNIFILTER™, Cat. No. 7700-1801 having a hydrophilic PVDF filter with an 800 $\mu$l/well capacity), which was placed on top of a 96-well box.
3. To obtain more proteins, 100-500 $\mu$l of lysis buffer containing fresh protease inhibitors was added to the cell debris, and Steps 1 and 2 were repeated.
4. The combined cell lysate was spun through the filter plate into a cold and clean 96-well box for 10-30 min at 3000 rpm. The volume of filtered lysate in each well was roughly 200-1000 $\mu$l.
5. Meanwhile, the required amount of glutathione beads (roughly 10-50 $\mu$l of beads per sample) (Amersham, USA) was washed four times with cold lysis buffer without the protease inhibitors, and finally resuspended in 5X of its original volume with lysis buffer containing fresh protease inhibitors.
6. 100 $\mu$l of washed glutathione beads was added to each well and sealed tightly with a cap mat. The beads were incubated with the lysate on a roller drum at 4°C for one hour. To obtain the best mixing, the boxes were rotated 360 degrees on the roller drum.
7. The beads were collected by spinning at 3000 rpm for 10-60 seconds, and the supernatant was discarded. Beads were washed once with 200-800 $\mu$l of Wash Buffer I containing protease inhibitors, and twice without the inhibitors.

8. The beads were then washed three times with 200-800 μl of Wash Buffer II. After complete removal of the buffer, 20-50 μl of Elution Buffer was added to each well. Filter plates used for the elution step comprised materials having low affinity for proteins (MILLIPORE MULTISCREEN™, Cat. No. MADVN6550 having a hydrophilic PVDF filter with a 200 μl/well capacity). The box was vortexed briefly to resuspend the beads and incubated on a roll drum for one hour at 4°C.

9. The eluate/beads slurry was transferred to a cold filter plate (Millipore, USA), and the eluate was collected to a 96-well PCR plate by spinning through the filter plate for 0.5-2 min at 3000 rpm at 4°C.

10. Each purified protein was aliquoted into three 96-well PCR plates and immediately stored in a -80°C freezer.

Lysis Buffer

| 30-300 mM | Tris pH 7.5 |
|---|---|
| 50-300 mM | NaCl |
| 0.1-10mM | EGTA |
| 0.01-1.0% | TritonX-100 |
| 0.01-1% | beta-mercaptoethanol ("BME") |
| 0.1-3 mM | phenylmethylsulfonyl fluoride ("PMSF") |

Roche Protease inhibitor tablets (containing EDTA)
BME, PMSF, and inhibitor tablets were added freshly.
Wash Buffer I:

| 30-300 mM | Tris pH 7.5 |
|---|---|
| 300-600 mM | NaCl |
| 0.1-10mM | EGTA |
| 0.01-1.0% | TritonX-100 |
| 0.01-1% | beta-mercaptoethanol ("BME") |
| 0.1-3 mM | PMSF |

Roche Protease inhibitor tablets (containing EDTA)
BME, PMSF, and inhibitor tablets were added freshly.
Wash Buffer II:

| 50-200 mM | HEPES pH 7.5 |
|---|---|
| 50-300 mM | NaCl |
| 1-15% | Glycerol |

Elution Buffer:

| 50-200 mM | HEPES pH 7.5 |
|---|---|
| 50-200 mM | NaCl |
| 20-40% | Glycerol |
| 5-40 mM | Glutathione (Reduced form) |

Elution buffer should be about pH=7.5.

**6.3 Method of Making Proteins in a High-Throughput 96-Array Format**

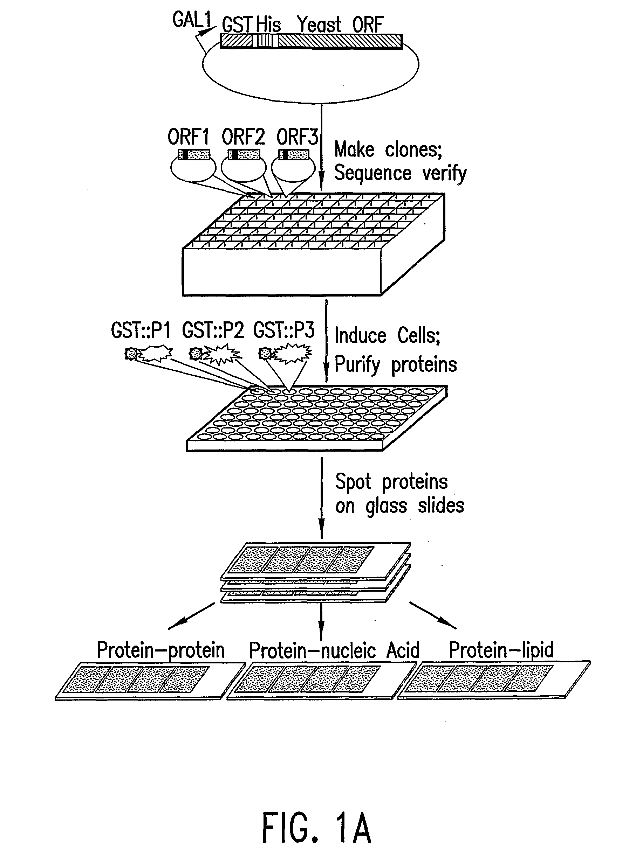

A yeast proteome microarray containing nearly all yeast proteins was prepared and screened for a number of biochemical activities. A high-quality collection of 5800 yeast ORFs (93.5% of the total) was cloned into a yeast high-copy expression vector using recombination cloning (Mitchell et al., 1993, Yeast 9:715). The yeast proteins were fused to GST-HisX6 at their amino termini and expressed in yeast under the control of a galactose-inducible *GAL1* promoter (Zhu et al., 2000, Nat. Genet. 26:283; Mitchell et al., 1993 Yeast 9:715). The yeast expression strains contain individual plasmids in which the correct yeast ORFs have been shown to be properly fused in-frame to GST by DNA sequencing. Briefly, yeast ORFs were amplified by PCR and co-transformed into yeast cells along with the vector to generate expression clones. The plasmids were rescued in *E. coli,* and the vector-insert junctions were sequenced (FIG. 1A). If the ORF cloned was not the ORF of interest, or a frameshift was detected, the cloning cycle was repeated. Once a construct was confirmed, the plasmid DNA was reintroduced into yeast and *E. coli* to create permanent stocks for future analyses (Zhu et al., 2000, Nat Genet. 26:283). By repeating the cloning cycle, 5800 unique yeast ORFs were successfully cloned, representing 93.5% of the total.

To generate purified proteins for biochemical analysis, a robust and high-throughput purification method for preparing proteins in a 96-well format was developed and optimized. Using glutathione-agarose beads, yeast extracts were prepared, and fusion proteins were purified (for details of 96-well format protein purification protocol, a full list of results from all the experiments, and the design of the positive identification algorithms, visit public web site spine.mbb.yale.edu/protein_chips/). The lysis buffer and initial washes contained 0.1 % Triton to ensure that the purified proteins were free of lipids. Using the procedures of the invention, at least 1152 protein samples can be prepared from cells in under 10 hours.

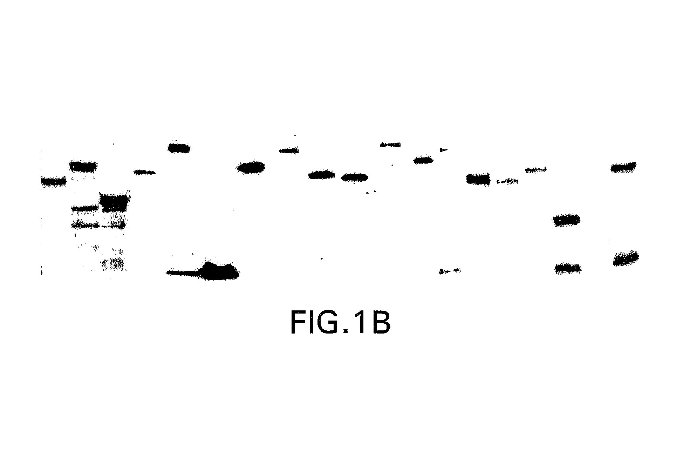

The quality and quantity of the purified proteins were monitored using immunoblot analysis of 60 random samples (FIG. 1B). Greater than 80% of the strains produced detectable amounts of fusion proteins of the expected molecular weight. A manual printing tool was used to spot 3 nl of 1152 purified proteins in duplicate onto glass slides (19), and the proteins detected using polyclonal anti-GST antibodies. Greater than 85% of the samples exhibited a visible signal above background, consistent with the immunoblot analysis. Using the procedures of the invention, fusion proteins from 6144 (64X96-well boxes) yeast strains can be purified in under two weeks.

### 6.4 Method of Making a Proteome Microarray

To prepare the proteome chips, 6566 protein preparations representing 5800 different yeast proteins were printed in duplicate onto glass slides using a commercially available microarrayer. As a control, known amounts of GST were also printed. Two types of slides were employed. Aldehyde-treated microscope slides were used in initial experiments (MacBeath et al., 2000, Science 289:1760), in which case fusion proteins were attached to the slide surface through primary amines at the N-termini or other residues of the fusion proteins, resulting in a relatively random orientation of proteins on the surface. Proteins were spotted onto nickel-coated slides in subsequent experiments. In this case, fusion proteins are attached through their HisX6 tags such that the cloned yeast portions of the fusion proteins are essentially uniformly oriented away from the surface. Although both types of slides were successfully used, the nickel-coated slides gave significantly superior signals for our particular protein preparations (FIG. 2A).

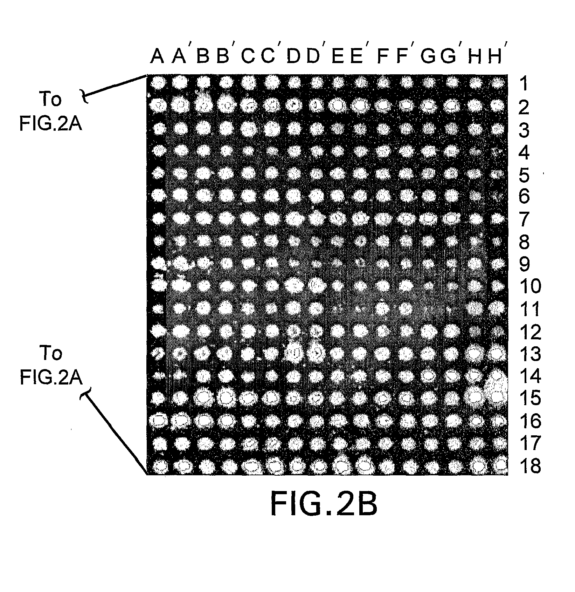

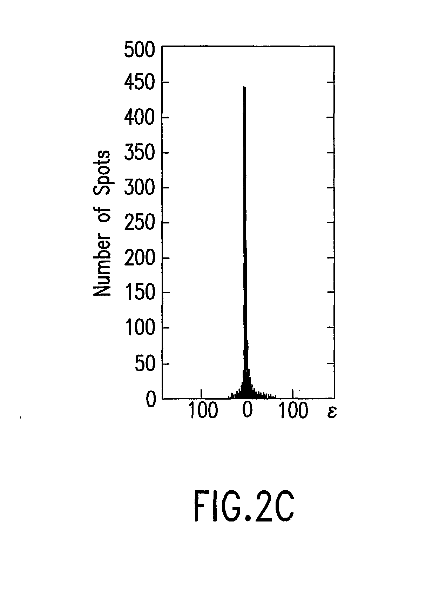

To determine how much fusion protein was covalently attached to different glass surfaces, and assess the reproducibility of the protein attachment, chips were probed with anti-GST antibodies. Over 93.5% of the protein samples gave signals significantly above background (*i.e.,* greater than approximately 10 fg of protein). A comparison with known amounts of GST also printed on the slide, indicated that about 90% of the spots contain approximately 10 fg to 950 fg of protein. FIG. 2A shows that detection of proteins on a proteome chip with fluorescently labeled antibodies is extremely sensitive, *i.e.,* the signal-to-noise ratio is high despite that only 1/10,000 of purified proteins from a 3-ml culture is spotted on the slide. The results demonstrate that it is feasible to spot 13,000 protein samples in one half the area of a standard microscope slide (2.5 cm by 7.5 cm) with excellent resolution (FIGS. 2A and 2B). To test the reproducibility of the protein spotting, the signals from each pair of duplicated spots were compared with one another. As shown demonstrated by the sharp spike in FIG. 2C, 95% of the signals were within 5% of the average (*10*).

### 6.5 Method of Using a Proteome Microarray

Proteome chips were tested by probing for several exemplary types of biological activities: protein-protein interactions, protein-nucleic acid interactions, and protein-lipid interactions.

Generally, proteome chips were prepared for assays as follows. The proteome chips were blocked by slowly immersing the printed glass slides into either BSA (1-3% (w/w) BSA in PBS buffer; SIGMA™, USA) or glycine blocking buffer (30-300 mM glycine; 50-300 mM Tris, pH 6.5-8.5; 50-300 mM NaCl; SIGMA™, USA) with the protein side up. The buffer was filtered through a 2 $\mu$m filter unit to remove particles. Glycine is preferable when probing for carbohydrate-binding proteins. The slides were incubated in the blocking buffer at 4°C overnight without any shaking (disturbance of the blocking buffer may result in the protein streaks on the glass surface).

Probe proteins were generally prepared as follows. Yeast proteins were purified by affinity column using glutathione beads from 50 ml culture using standard protocols without the elution from the beads. The protein beads were washed three to five times with cold PBS buffer (pH 8.0) (SIGMA™, USA). Approximately 1 ml of Sulfo-NHS-LC-LC-Biotin (PIERCE™ Cat No. 21338, USA) dissolved in PBS (pH 8.0) at a concentration of 0.1-50 mg/ml was added to the glutathione beads and incubated at 4°C for 2h. The beads were washed 5 times with cold PBS buffer (pH 8.0) and eluted with 100-500 ml of the elution buffer (50-200 mM, HEPES pH 7.5; 50-200 mM NaCl; 20-40% glycerol; 5-40 mM glutathione). Protocols resulting in more weakly biotinylated proteins are preferred. Batches of proteins that are biotinylated to different degrees were pooled for future usage.

### 6.5.1 Identification of Calmodulin-Interacting Proteins

To test for protein-protein interactions, the yeast proteome was probed with calmodulin (11). Calmodulin is a highly conserved calcium-binding protein involved in many calcium-regulated cellular processes and has many known physical partners (Hook et al., 2001, Annu. Rev. Pharmacol. Toxicol. 41:471). The calmodulin probe was biotinylated, and bound probe was detected using Cy3-labeled streptavidin. As a control, the yeast proteome was probed with Cy3-labeled streptavidin alone.

Generally, protein-protein interactions can be assayed as follows. Blocked proteome chips are washed three to five times in PBS buffer, and the extra liquid on the glass surface is removed by tapping the slides vertically on a KIMWIPE™. 200 μl of biotinylated protein probe is added to the proteome chip and immediately covered by a hydrophobic plastic cover slip (GRACE BIO-LABS™, USA). After trapped air bubbles are removed, the chip is incubated in a humidity chamber at room temperature (RT) for one hour. To remove the cover slip, the chip is immersed into large volume of PBS buffer (>50 ml), and the slip should float away. The chip is then moved to a second PBS bath (>50 ml) and washed 3 X 5 min with shaking at RT. After removing extra liquid on the surface of the chip, at least 150 μl of Cy3- or Cy5-conjugated streptavidin (PIERCE™, USA; 1:2000 to 1:4000 dilution) is added to the surface and covered by a hydrophobic plastic cover slip (GRACE BIO-LABS™, USA). The chip is incubated for greater than 30 min in the dark at RT. The chip is then washed as described above. To completely remove the liquid on the chip, the chip is spun to dryness at 1500-2000 rpm for 5-10 min at RT.

If a proteome chip is to be screened with antibodies, the protein-antibody interaction can be detected as follows. Blocked proteome chips are washed three to five times in PBS buffer, and the extra liquid on the glass surface is removed by tapping the slides vertically on a KIMWIPE™. 200 μl of primary antibodies (properly diluted in PBS containing 1-3% BSA and 0.1% TritonX-100) is added to the proteome chip and immediately covered by a hydrophobic plastic cover slip (GRACE BIO-LABS™, USA). After trapped air bubbles are removed, the chip is incubated in a humidity chamber at RT for one hour. To remove the cover slip, the chip is immersed into large volume of PBS buffer (>50 ml), and the slip should float away. The chip is then moved to a second PBS bath (>50 ml) and washed 3 X 5 min with shaking at RT. After removing extra liquid on the surface of the chip, >150 μl of Cy3- or Cy5-conjugated secondary antibodies (properly diluted in PBS containing 1-3% BSA and 0.1% TritonX-100) is added to the surface and covered by a hydrophobic plastic cover slip (GRACE BIO-LABS™, USA). The chip is incubated for greater than 30 min in the dark at RT. The chip is then washed as described above. To completely remove the liquid on the chip, the chip is spun to dryness at 1500-2000 rpm for 5-10 min at RT.

When biotinylated calmodulin was used to probe the proteome chips in the presence of calcium, six known calmodulin targets, namely Cmk1p, Cmk2p, Cmp2p, Dst1p, Myo4p, and Arc35p were identified (FIG. 3A). Cmk1p and Cmk2p are type I and II calcium/calmodulin-dependent serine/threonine protein kinases, which are both involved in the signal transduction pathway in the mating response (Hook et al., 2001, Annu. Rev. Pharmacol. Toxicol. 41). Cmp2p (Cna2p) is one of the two yeast calcineurins, and has been demonstrated to interact with calmodulin in vivo (Cyert et al., 1991, Proc. Natl. Acad. Sci. U.S.A. 88:7376), Dstlp plays a role in transcription elongation (Stirling et al., 1994, EMBO J. 13:4329), Myo4p is a class V myosin heavy chain required for proper localization of ASH1 transcript (Bohl et al., 2000, EMBO J. 19:5514; Bertrand et al., 1998, Mol. Cell 2:437). Arc35p is a component of the Arp2/3 actin-organizing complex, which is involved in actin assembly and function, and in endocytosis (Winter et al., 1999, Proc. Nat. Acad. Sci. U.S.A. 96:7288). Arc35p was recently shown to interact with calmodulin in a two-hybrid study (Schaerer-Brodbeck et al., 2000, Mol. Biol. Cell 11:1113), thus confirming the data herein demonstrating that Arc35p and calmodulin interact in vitro. Of the six known calmodulin targets that were not detected, two were not represented in the collection and the remaining four were not detectable in the GST probing experiments. In addition to known interactors, the calmodulin probe identified 33 additional interactors. These interactors include a wide variety of different types of proteins (see Table 1; public web site bioinfo.mbb.yale.edu/proteinchip), consistent with a role for calmodulin in many diverse cellular processes.

In addition to the calmodulin-binding targets, one protein, Pyc1p, which bound Cy3-labeled streptavidin, was also identified. Pyc1p encodes a pyruvate carboxylase 1 homolog that contains a highly conserved biotin-attachment region (Menendez et al., 1998, Yeast 14:647). Thus, as predicted by its sequence, Pyc1p is biotinylated in vivo; and was identified in all experiments using streptavidin detection methods. Thus, proteome microarrays can be used to identify posttranslational modifications of proteins.

**6.5.2 Identification of a Calmodulin-binding Motif**

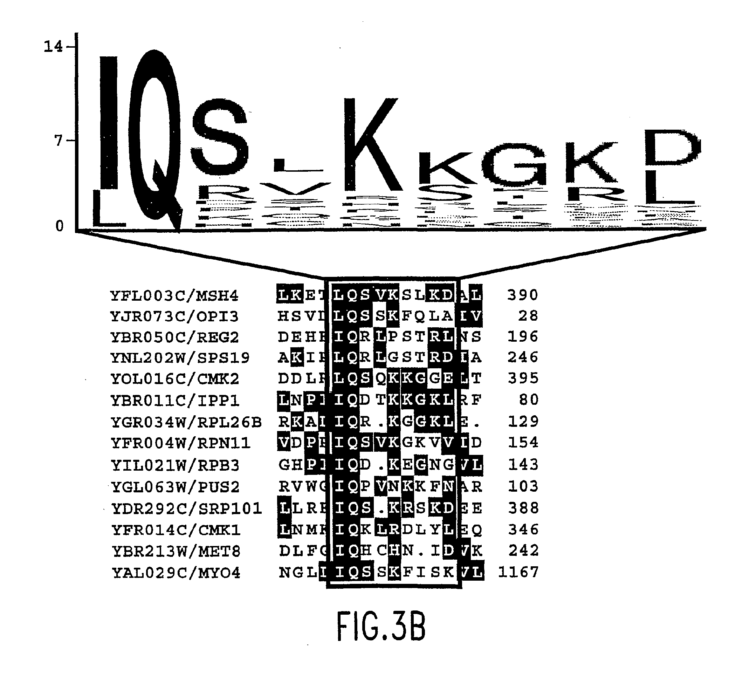

To identify putative calmodulin-binding domains, amino acid sequences shared among the different calmodulin-binding targets (*i.e.,* interactors) were determined (Zhu et al., 2000, Nat. Genet. 26:283). Fourteen of the 39 calmodulin-binding proteins contain a motif whose consensus is I/L-Q-X-X-K-K/X-G-B (SEQ ID NO: 1), where X is any residue and B is a basic residue (FIG. 3B). A related sequence in myosins, I-Q-X-X-X-X-K-X-X-X-R (SEQ ID NO: 16), has been shown previously to bind calmodulin (Homma et al., 2000, J. Biol. Chem 275). The results demonstrate that the motif, I-Q-X-X-X-X-K-X-X-X-R (SEQ ID NO: 16), is found in many calmodulin-binding proteins. Other calmodulin-binding interactors that lack this motif can possess other calmodulin-binding sequences.

**6.5.3 Identification of a ATP/GTP-binding Proteins**

1. Blocked proteome chips are washed three to five times in cold PBS buffer, and the extra liquid on the glass surface is removed by tapping the slides vertically on a KIMWIPE™.

2. 100 μl of ATP and GTP solution (0.5-5 mM ATP-Cy3, 0.5-5mM GTP-Cy5, 100-400 mM NaCl, 1-30 mM $MgCl_2$, 50-300 mM Tris, pH 7-8.5) is added to the proteome chip and immediately covered by a hydrophobic plastic cover slip (GRACE BIO-LABS™, OR, USA). After trapped air bubbles are removed, the chip is incubated in a humidity chamber at 4°C for one hour.

3. To remove the cover slip, the chip is immersed into large volume (>30 ml) of ice cold wash buffer (100-400 mM NaCl, 1-30 mM $MgCl_2$, 50-300 mM Tris, pH 7-8.5), and the cover slip should float away. The chip is then moved to a second cold wash bath (>50 ml) and washed 3 X 5 min with shaking at 4°C.

4. After removing extra liquid on the surface of the chip, the chip is spun to dryness at 1500-2000 rpm for 5-10 min at 4°C.

**6.5.4 Identification of DNA-binding Proteins**

Protein-DNA and protein-RNA interactions are important for many fundamental biological functions such as transcription regulation, chromosome segregation and maintenance, and RNA transport and processing (Williamson, 2000, Nat. Struct. Biol. 7(10):834-7). To explore the possibility of using a proteome chip to identify proteins that bind to DNA or RNA molecules, total yeast genomic DNA was labeled using Cy3-CTP and Klenow fragment, and polyA+ was labeled using a biotinylated psoralen-derivative (31). Each probe was incubated with a different proteome chip (FIG. 3A).

The protein-nucleic acid interaction was assayed as follows. The nucleic acid probes were labeled as described by Winzeler et al. (1999, Science 285:901). Blocked proteome chips were washed three to five times in PBS buffer, and the extra liquid on the glass surface was removed by tapping the slides vertically on a KIMWIPE™. 200 μl of labeled nuclei acid probe (50-200 mM poly(dC/dG); 100-300 mM NaCl; 50-300 mM HEPES, pH 7.0-8.5; 10 mM $MgCl_2$) was added to the proteome chip and immediately covered by a hydrophobic plastic cover slip (GRACE BIO-LABS™, USA). After trapped air bubbles were removed, the chip was incubated in a humidity chamber in the dark at room temperature ("RT") for one hour. To remove the cover slip, the chip was immersed into large volume of PBS buffer(>50 ml), so that the slip would float away. The chip was then moved to a second PBS bath (>50 ml) and washed 3 X 5 min with shaking in the dark at RT. To completely remove the liquid on the chip, the chip was spun to dryness at 1500-2000 rpm for 5-10 min at RT.

Visual inspection revealed that the genomic DNA probe identified 41 DNA-binding proteins (*see* Table 1). These included eight previously known DNA-associated proteins and two predicted DNA-binding proteins. Of the known DNA-binding proteins, we found three DNA-repair proteins (*i.e.,* xrs2p, Cdc1p, and Rad51p (Costanzo et al., 2001, Nucleic Acids Res. 29:75)), a SWI-SNF global transcription activator complex component (*i.e.,* Snfl 1p (Costanzo et al. 2001, Nucleic Acids Res. 29:75)), the alpha-subunit of the NC2 (Dr1/Drap1) repressor of class II transcription Bur6p (Sternberg. et al., 1987, Cell 48:567), the transcription factor Spt2p (a negative regulator of HO gene transcription (Sternberg et al., 1987, Cell 48:567)), and Met30p which contains five WD-repeats and binds certain promoter regions (Thomas et al., 1995, Mol. Cell Biol. 15:6526). We also identified Trm2p, which binds both DNA and RNA and is involved in DNA repair and RNA processing/modification (Sadekova et al., 1996, Curr. Genet. 30(1):50-5.).

Eight proteins, identified as nucleic-acid binding proteins, have unknown functions. Of these, three are likely to possess DNA-binding activities based on their amino acid sequences and the results of the instant assay. Ycr087c contains a zinc-finger domain, a common DNA-binding motif. Yhr054c shares sequence homology with the transcription factor Rsc3p (Costanzo et al., 2001, Nucleic Acids Res. 29:75). Ybr025c is believed to have putative nucleotide-binding activity (Costanzo et al., 2001, Nucleic Acids Res. 29:75). The instant assay shows that these eight proteins bind DNA *in vitro* and likely bind nucleic acids *in vivo.*

**TABLE 1**

| ORF | Block | Column | Row | PC | PI(3,4,5)P$_3$ | PI(3,4)P$_2$ | PI(3)P | PI(4,5)P$_2$ | PI(4)P |
|-----|-------|--------|-----|-----|-----|-----|-----|-----|-----|
| "-" indicates spots that are flagged as bad, which were given the value -10000 in the data table. | | | | | | | | | |
| MPS1 | 7 | 13 | 17 | 4374.59 | 1864.12 | 4216.40 | 310.00 | 1084.79 | 6148.60 |
| YGR052W | 13 | 13 | 17 | 2177.02 | 666.22 | 2328.28 | 9.00 | 563.52 | 164.18 |
| YLL023C | 47 | 7 | 18 | 1798.73 | 635.18 | 390.40 | - | 1868.44 | 1712.76 |
| SNF1 | 41 | 15 | 17 | 1774.37 | 560.70 | 298.26 | 202.00 | 423.73 | 1795.06 |
| YFL068W | 11 | 9 | 4 | 1541.05 | 369.22 | 890.06 | 82.75 | 190.96 | 931.28 |
| GCN2 | 25 | 15 | 17 | 1087.04 | 975.60 | 232.01 | 26.50 | 505.60 | 1356.97 |
| YFR053C | 7 | 13 | 4 | 1077.95 | 235.69 | 713.77 | 238.75 | 613.81 | 991.38 |
| CMK1 | 13 | 15 | 17 | 1073.54 | 699.07 | 1786.71 | 7.00 | 545.23 | 105.39 |
| YGL059W | 21 | 15 | 17 | 970.24 | 273.38 | 3088.36 | 91.50 | 299.61 | 924.10 |
| CAK1 | 41 | 13 | 17 | 854.02 | 269.10 | 245.20 | 99.50 | 233.85 | 1431.44 |
| BUD32 | 45 | 13 | 17 | 621.00 | 108.67 | 249.28 | 318.00 | 282.63 | 666.29 |
| SPS1 | 17 | 13 | 17 | 557.02 | 389.70 | 1147.50 | 88.50 | 358.84 | 246.92 |
| TOS3 | 37 | 15 | 17 | 535.89 | 623.47 | 766.36 | 167.00 | 685.45 | 2026.31 |
| APG1 | 45 | 15 | 17 | 464.28 | 92.70 | 138.45 | 72.00 | 319.65 | 668.47 |
| LYS1 | 14 | 1 | 6 | 255.33 | 61.20 | 81.94 | 3.50 | 22.21 | 198.15 |
| YHR174W | 34 | 5 | 6 | 250.34 | 80.55 | 274.24 | 19.25 | 47.69 | 192.48 |
| OPY1 | 23 | 15 | 1 | 250.04 | 41.17 | 179.27 | 17.50 | 37.89 | 188.13 |
| RIM15 | 5 | 15 | 17 | 244.76 | 1219.28 | 4941.00 | 52.50 | 687.19 | 2349.00 |
| YBR028C | 3 | 13 | 17 | 232.43 | 147.60 | 225.42 | 18.00 | 165.05 | 209.03 |
| YCL075W | 32 | 11 | 2 | 221.87 | 115.88 | 192.45 | 51.50 | 116.71 | 288.29 |
| SLS1 | 17 | 7 | 6 | 220.99 | *166.05* | 230.60 | 17.00 | -4.57 | - |
| COX20 | 24 | 5 | 3 | 214.24 | 14.06 | 199.20 | 35.25 | 26.56 | 153.94 |
| AKL1 | 11 | 13 | 17 | 207.20 | 93.38 | 132.49 | 19.50 | 57.48 | 189.44 |
| HIS7 | 18 | 3 | 2 | 193.11 | 78.07 | - | 20.00 | 18.73 | 226.45 |
| MET8 | 47 | 13 | 1 | 188.12 | 25.42 | 95.44 | 117.00 | 244.09 | 175.94 |
| YMR172C-A | 4 | 1 | 16 | 174.33 | 94.05 | 264.35 | 28.00 | 101.47 | 109.31 |
| PAT1 | 30 | 7 | 17 | 160.24 | 56.47 | 10.05 | - | 66.63 | 158.95 |
| YEL048C | 2 | 11 | 4 | 140.58 | 87.75 | 76.13 | 17.75 | 45.07 | 68.81 |
| GPM3 | 44 | 7 | 10 | 137.64 | 66.04 | 85.40 | 42.50 | 112.57 | 85.35 |
| YOL117W | 44 | 5 | 10 | 134.71 | 67.95 | 78.02 | -0.50 | 123.90 | 88.84 |
| DFG5 | 26 | 5 | 9 | 127.66 | 60.98 | 48.66 | - | - | 19.81 |
| YFR020W | 25 | 9 | 4 | 127.37 | 33.86 | 26.53 | 0.75 | 49.43 | 111.70 |
| KCC4 | 7 | 15 | 17 | 123.26 | 65.47 | 234.84 | 33.50 | 67.06 | 84.92 |
| ROT1 | 2 | 9 | 9 | 121.50 | 99.11 | 137.98 | 33.25 | 63.58 | 52.04 |
| YDR288W | 11 | 11 | 3 | 121.50 | 97.20 | 220.55 | 24.00 | 40.94 | 183.34 |
| BTT1 | 38 | 13 | 3 | 120.33 | 37.12 | 28.26 | - | - | 135.44 |

(continued)

| ORF | Block | Column | Row | PC | PI(3,4,5)P$_3$ | PI(3,4)P$_2$ | PI(3)P | PI(4,5)P$_2$ | PI(4)P |
|---|---|---|---|---|---|---|---|---|---|
| "-" indicates spots that are flagged as bad, which were given the value -10000 in the data table. | | | | | | | | | |
| ARP10 | 34 | 13 | 3 | 119.74 | 30.15 | 29.20 | 51.00 | 32.01 | 98.64 |
| YLR022C | 47 | 9 | 18 | 118.57 | 21.38 | 15.54 | - | 152.85 | 219.92 |
| YDR070C | 2 | 5 | 3 | 109.17 | 48.26 | 140.65 | 34.75 | 67.28 | 36.15 |
| YBL107W-A | 1 | 15 | 1 | 108.59 | 37.12 | 128.72 | 15.50 | - | 12.63 |
| FBP26 | 21 | 9 | 4 | 107.41 | 25.31 | 48.51 | 5.75 | 47.90 | 62.06 |
| MY04 | 6 | 15 | 17 | 103.89 | 51.07 | 171.73 | 2.00 | 4.79 | 36.58 |
| YER152C | 22 | 5 | 4 | 103.60 | 50.17 | 24.80 | -3.75 | 20.47 | 104.95 |
| FUR1 | 4 | 11 | 6 | 95.67 | 72.22 | 210.19 | 112.25 | 111.70 | 148.94 |
| YPL280W | 28 | 7 | 17 | 88.63 | 43.88 | 30.14 | - | - | 118.45 |
| YBR077C | 17 | 13 | | 86.58 | - | 129.98 | 4.75 | 21.12 | 66.85 |
| TAT2 | 12 | 7 | 10 | 83.93 | 68.29 | 62.48 | 60.50 | 85.79 | 51.39 |
| YIR041W | 22 | 5 | 6 | 75.72 | 130.39 | 38.15 | 6.50 | 6.53 | 200.10 |
| YKL031W | 32 | 11 | 18 | 75.72 | 39.71 | 69.38 | 36.25 | 58.14 | 165.48 |
| YGR242W | 25 | 3 | 5 | 75.13 | 35.77 | 11.93 | 17.00 | 10.02 | 38.32 |
| HOR2 | 34 | 13 | 4 | 74.84 | 4.16 | -3.30 | 11.00 | 7.19 | 68.15 |
| TAF25 | 4 | 7 | 17 | 73.37 | 54.67 | 110.51 | -10.00 | 77.08 | 45.29 |
| YJL193W | 33 | 13 | 6 | 72.78 | - | 21.03 | 97.00 | 27.65 | 103.43 |
| MIS1 | 16 | 7 | 17 | 71.02 | 43.65 | 86.34 | 3.50 | - | 65.32 |
| PEX4 | 16 | 5 | 5 | 69.85 | 21.49 | 8.63 | 1.75 | 28.31 | 57.48 |
| SUN4 | 46 | 9 | 9 | 69.55 | 33.19 | 20.88 | - | 66.41 | 60.75 |
| YOL073C | 12 | 5 | 10 | 68.67 | 17.32 | 41.44 | 1.75 | 26.56 | 40.06 |
| A1 | 4 | 3 | 16 | 68.38 | 48.49 | 88.38 | 31.75 | 46.81 | 31.57 |
| YML058C-A | 3 | 5 | 8 | 66.33 | 40.16 | 76.29 | 20.50 | 50.52 | 34.19 |
| RPG1 | 17 | 9 | 1 | 66.33 | 22.05 | 61.53 | -2.25 | -6.75 | 54.00 |
| VTC4 | 40 | 9 | 6 | 66.03 | 25.31 | 30.45 | 8.50 | 26.78 | 99.07 |
| YDR149C | 16 | 11 | 3 | 65.74 | 24.86 | 73.62 | 37.50 | 37.02 | 46.81 |
| FRM2 | 2 | 15 | 17 | 63.39 | 16.88 | 59.34 | 3.50 | 75.34 | 12.63 |
| WRS1 | 26 | 5 | 10 | 62.51 | 51.08 | 63.10 | - | 21.56 | 4.57 |
| DPS1 | 47 | 15 | 18 | 62.22 | 13.05 | 25.12 | - | 41.81 | 99.29 |
| PKR1 | 47 | 5 | 8 | 61.63 | 15.75 | 37.52 | - | 87.75 | 61.40 |
| YDR067C | 2 | 9 | 3 | 61.34 | -29.47 | 29.83 | 32.75 | 47.90 | 17.42 |
| YCR025C | 8 | 5 | 2 | 60.46 | 73.58 | 174.24 | 60.00 | 68.37 | 54.87 |
| APS2 | 47 | 15 | 6 | 58.70 | 15.97 | 56.51 | 39.50 | 103.65 | 159.82 |
| RRP5 | 48 | 5 | 16 | 58.11 | 21.26 | 22.60 | - | 73.38 | 60.31 |
| YMR148W | 12 | 7 | 9 | 56.93 | 50.17 | 53.69 | 36.50 | 43.33 | 23.08 |
| RPH1 | 14 | 13 | 2 | 56.93 | 9.90 | 90.73 | 6.00 | 18.73 | 66.19 |
| RGD1 | 36 | 15 | 2 | 54.59 | 19.35 | 54.31 | 6.00 | 55.31 | 127.60 |

(continued)

| "-" indicates spots that are flagged as bad, which were given the value -10000 in the data table. | | | | | | | | | |
|---|---|---|---|---|---|---|---|---|---|
| ORF | Block | Column | Row | PC | PI(3,4,5)P$_3$ | PI(3,4)P$_2$ | PI(3)P | PI(4,5)P$_2$ | PI(4)P |
| YMR244W | 44 | 5 | 9 | 54.29 | 28.24 | 29.20 | 64.75 | 44.20 | 32.66 |
| SAS3 | 6 | 3 | 17 | 54.00 | 18.68 | 55.57 | 2.00 | 17.85 | 16.55 |
| SGA1 | 16 | 3 | 16 | 52.83 | 26.55 | 160.59 | 16.50 | 6.75 | 41.15 |
| SXM1 | 30 | 11 | 13 | 51.95 | 23.40 | 19.15 | - | 27.22 | 30.48 |
| MUM2 | 3 | 13 | 1 | 46.66 | 63.34 | 49.29 | 81.50 | 38.76 | 90.15 |
| VMA13 | 4 | 5 | 17 | 45.78 | 28.12 | 44.27 | 11.50 | 34.40 | 16.55 |
| YJL185C | 9 | 11 | 5 | 45.49 | 50.96 | 127.47 | -3.25 | 13.94 | 27.00 |
| YOL119C | 44 | 1 | 10 | 44.61 | 14.62 | 5.97 | 360.50 | 23.08 | 22.65 |
| SPS2 | 47 | 11 | 3 | 44.61 | 25.31 | 54.63 | 18.50 | 64.02 | 47.47 |
| YBL107C | 3 | 15 | 1 | 44.02 | 65.93 | 23.86 | 104.50 | 34.84 | 40.50 |
| THI13 | 2 | 11 | 3 | 43.14 | 39.04 | 75.66 | 13.75 | 64.67 | 55.09 |
| KCS1 | 16 | 5 | 17 | 42.85 | 36.22 | 60.91 | 0.00 | - | 35.27 |
| APA2 | 16 | 15 | 16 | 42.26 | 40.05 | 31.40 | 22.50 | 34.84 | 43.98 |
| HYS2 | 7 | 3 | 6 | 41.97 | 33.41 | 70.80 | 3.50 | 15.90 | 50.30 |
| DRS1 | 39 | 7 | 18 | 39.62 | 11.02 | 10.67 | 0.25 | 20.69 | 35.71 |
| GPA2 | 44 | 3 | 4 | 39.33 | 13.05 | 119.93 | -50.50 | 44.85 | 87.31 |
| SLT2 | 3 | 13 | 13 | 38.15 | 90.90 | 93.09 | 26.25 | 24.39 | 32.88 |
| HEM14 | 20 | 15 | 16 | 38.15 | 8.55 | 21.03 | 1.00 | - | 16.11 |
| TFB3 | .43 | 13 | 3 | 37.86 | 27.45 | 47.72 | 71.00 | 37.23 | 43.98 |
| PCL6 | 29 | 5 | 1 | 31.70 | 26.89 | 26.84 | 9.00 | 3.70 | 8.06 |
| YDL237W | 4 | 15 | 3 | 31.10 | 39.15 | 46.47 | 132.50 | 40.06 | 31.35 |
| YHL041W | 41 | 1 | 5 | 31.11 | 21.15 | 19.78 | 4.00 | 15.24 | 101.90 |
| YOL054W | 44 | 11 | 10 | 29.05 | 19.12 | 32.81 | -15.50 | 47.90 | 28.74 |
| YPL150W | 47 | 7 | 17 | 28.76 | 16.20 | 0.31 | - | 34.84 | 38.76 |
| ARO4 | 28 | 15 | 2 | 28.17 | 29.25 | 34.53 | 87.50 | 20.47 | 42.24 |
| YGR176W | 48 | 5 | 5 | 26.12 | -4.05 | -2.83 | - | 16.11 | 32.88 |
| MPD2 | 20 | 1 | 10 | 25.24 | 15.07 | 31.71 | - | 8.71 | 37.45 |
| GAD1 | 34 | 5 | 9 | 24.36 | - | - | 51.25 | 11.54 | -1.96 |
| DTR1 | 24 | 15 | 17 | 24.07 | 13.72 | 2.83 | 23.00 | 3.05 | -4.79 |
| ARG1 | 44 | 3 | 10 . | 23.77 | -6.08 | 1.73 | 213.00 | 3.48 | -6.31 |
| PAU5 | 17 | 15 | 4 | 22.30 | - | -5.02 | -3.00 | -1.31 | 31.35 |
| CCT8 | 30 | 1 | 6 | 22.30 | 33.97 | 7.53 | - | - | 23.52 |
| YPL275W | 48 | 13 | 10 | 21.72 | 2.59 | 9.89 | - | 237.77 | 14.37 |
| DIN7 | 30 | 5 | 3 | 21.42 | 14.06 | 10.05 | - | 14.59 | 33.75 |
| URA8 | 29 | 13 | 6 | 20.25 | 42.86 | -2.83 | 11.50 | 23.95 | 34.40 |
| YPL107W | 28 | 15 | 11 | 18.78 | 14.62 | 27.00 | 21.50 | 10.45 | 5.23 |
| CAM1 | 47 | 13 | 10 | 18.49 | 4.16 | -0.16 | - | 25.69 | 21.99 |

(continued)

| "-" indicates spots that are flagged as bad, which were given the value -10000 in the data table. | | | | | | | | | |
| --- | --- | --- | --- | --- | --- | --- | --- | --- | --- |
| ORF | Block | Column | Row | PC | PI(3,4,5)P$_3$ | PI(3,4)P$_2$ | PI(3)P | PI(4,5)P$_2$ | PI(4)P |
| KSS1 | 5 | 13 | 17 | 16.43 | 0.23 | 87.91 | -11.50 | -2.61 | 10.45 |
| PET9 | 30 | 3 | 1 | 14.97 | 17.66 | -5.81 | - | 26.13 | - |
| YDR348C | 41 | 15 | 3 | 14.67 | 27.00 | 53.69 | 43.50 | 25.69 | 57.92 |
| KGD2 | 48 | 1 | 3 | 14.09 | 12.60 | 19.78 | - | 69.68 | 60.97 |
| YGL165C | 12 | 7 | 5 | 13.50 | 16.65 | 16.80 | 8.50 | 18.73 | 16.33 |
| SSB1 | 39 | 15 | 16 | 12.91 | 4.27 | 6.28 | - | 1.31 | 16.98 |
| MAG1 | 14 | .9 | 4 | 10.86 | 29.02 | -2.20 | 6.50 | 5.23 | 21.34 |
| ASF1 | 37 | 15 | 13 | 10.57 | -13.05 | 1.57 | 49.00 | 14.81 | 5.66 |
| YKL121W | 37 | 13 | 13 | 10.27 | 29.92 | 7.53 | 66.00 | 16.33 | -1.52 |
| YOL053W | 42 | 15 | 10 | 7.63 | 7.65 | 5.34 | - | 5.66 | 29.18 |
| YLR020C | 43 | 13 | 7 | 7.34 | 1.24 | 2.67 | 3.75 | 15.68 | -5.66 |
| PYK1 | 34 | 15 | 1 | 6.46 | -1.12 | -5.02 | -9.00 | -13.06 | 10.45 |
| RPL9B | 48 | 5 | 9 | 6.46 | -0.45 | 7.38 | - | 1.96 | 12.85 |
| APG7 | 34 | 15 | 6 | 5.87 | 0.45 | -0.63 | -22.50 | 59.23 | 18.29 |
| SPO1 | 6 | 5 | 9 | 5.58 | -9.22 | 18.99 | -6.50 | 14.37 | 0.44 |
| RTG3 | 48 | 5 | 1 | 5.58 | -5.29 | 0.16 | - | 12.41 | 12.19 |
| YEL064C | 12 | 15 | 4 | 5.28 | -0.90 | -3.14 | -5.00 | 6.53 | -3.92 |
| BPL1 | 48 | 7 | 17 | 5.28 | 8.77 | 6.59 | - | 22.21 | 25.26 |
| GFD1 | 42 | 9 | 9 | 4.99 | 3.38 | -0.78 | - | - | -0.22 |
| YJR080C | 7 | 1 | 6 | 3.52 | 32.17 | 78.17 | -1.50 | 13.50 | 32.66 |
| YGR101W | 40 | 15 | 5 | 3.52 | -4.05 | - | 4.50 | -11.76 | -6.97 |
| MRP1 | 43 | 15 | 3 | 3.52 | 13.95 | 18.84 | 54.50 | 21.34 | 23.52 |
| YNL051W | 40 | 9 | 9 | 3.23 | 25.31 | 21.19 | - | 35.06 | 34.40 |
| STP22 | 28 | 13 | 2 | 2.93 | 22.05 | 8.79 | 51.50 | -8.27 | 10.89 |
| MUB1 | 29 | 9 | 8 | 2.64 | 9.79 | 9.89 | -6.00 | -4.79 | 19.38 |
| ATP1 | 48 | 13 | 1 | 0.29 | 26.44 | 11.93 | - | 46.38 | 28.09 |
| CSE4 | 44 | 11 | 7 | -0.88 | -8.66 | -8.95 | -47.75 | -8.06 | 2.61 |
| VAC7 | 40 | 5 | 9 | -1.17 | 13.16 | 19.62 | 28.75 | 21.99 | 28.74 |
| GFA1 | 38 | 9 | 18 | -1.76 | -6.53 | -8.63 | - | -7.62 | 3.05 |
| SDS22 | 43 | 11 | 18 | -4.70 | -2.14 | 4.71 | 2.00 | 14.15 | 13.28 |
| YER046W | 18 | 9 | 4 | -18.20 | 5.62 | 11.62 | - | 6.31 | 19.16 |
| YBR226C | 2 | 11 | 2 | - | 55.35 | 65.93 | 42.00 | 87.97 | 56.61 |
| YBR025C | 17 | 3 | 1 | - | -39.15 | -11.93 | 0.50 | 8.93 | -9.15 |
| RFA3 | 17 | 5 | 6 | - | 71.10 | 106.12 | 5.25 | 3.27 | 32.66 |
| MHR1 | 19 | 15 | 3 | - | 14.40 | 35.48 | 12.00 | 10.89 | 70.89 |
| ERV1 | 34 | 9 | 5 | - | 1.35 | - | 15.75 | 18.94 | -3.27 |
| TOS6 | 37 | 3 | 9 | - | 34.31 | 15.85 | -5.00 | 16.11 | 41.81 |

(continued)

| "-" indicates spots that are flagged as bad, which were given the value -10000 in the data table. | | | | | | | | | |
| --- | --- | --- | --- | --- | --- | --- | --- | --- | --- |
| ORF | Block | Column | Row | PC | PI(3,4,5)P$_3$ | PI(3,4)P$_2$ | PI(3)P | PI(4,5)P$_2$ | PI(4)P |
| YNR069C | 37 | 5 | 9 | - | 18.79 | 4.55 | -13.75 | 9.36 | 28.74 |
| SDS3 | 42 | 15 | 4 | - | 9.67 | 3.45 | - | 4.35 | 11.76 |
| YIL00W | 42 | 13 | 6 | - | 14.29 | 15.54 | - | 34.40 | 36.58 |

### 6.5.5 Identification of RNA-binding Proteins

In addition to Trm2p, other interactors with RNA-binding activity were identified. For example, an isoleucyl-tRNA synthetase (Ils1p) and a ribosomal protein (Rp141B) have both been shown to bind RNA molecules (Lanker et al., 1992, Cell 70:647; Suzuki et al., 1990, Curr. Genet 17:185). Other proteins involved in translation tested positive in the screen for RNA-binding proteins such as Rp141b, Sqt1p, and Gcd1 1p. Rp141b is a component of the large ribosomal subunit. Sqt1p interacts with Rp110p, which is an RNA-binding protein (Costanzo, 2001, Nucleic Acids Res. 29:75). Gcd1 1p, a gamma subunit of translation initiation factor eIF2 has GTP-binding activity, and binds to the Lsm1-7 complex (U6-specific snRNP) as demonstrated by two-hybrid analysis (FromontRacine et al., 2000, Yeast 17:95). Two other protein targets that bind the DNA probe also bind nucleotides, *i.e.*, Thi13p is involved in nucleotide metabolism, and Ypt31p has GTP-binding activity (Costanzo et al., 2001, Nucleic Acids Res. 29:75). The RNA-binding and nucleotide-binding proteins identified with the DNA probe may reflect low affinity interactions with DNA or, alternatively, may represent normal affinity for DNA that has been previously unrecognized.

RNA-polyA$^+$ from exponentially growing cells was labeled with biotin and used to probe the proteome microarray. The positive signals were detected using Cy3-streptavidin. Nineteen target interactors were identified (*see* Table 1), including a U1 snRNP component (Smd1p), a protein known to bind mRNA import protein (Sxm8p), a C2-H2 zinc-finger protein (Azflp), and Tos8p, which was suggested by its sequence to be involved in RNA splicing and processing (Schwikowski et al., 2000, Nat. Biot. 18:1257). In addition to proteins that likely directly or indirectly interact with RNA, we found two transcription factors (Bur6p and Tos8p), one transcriptional silencer (Hst3p), six other proteins encoded by known ORFs, and seven proteins from uncharacterized ORFs. One of the unknown ORFs exhibited the highest signals (Yer152c). It is important to note that only one interactor, Bur6p, bound both the DNA and RNA probes. The other interactors were uniquely detected with either a double-stranded DNA probe or a RNA probe, suggesting that most proteins are likely to specifically bind either DNA or RNA.

In summary, of the 41 target interactors, eighteen are nucleic-acid interacting proteins; eleven are known or putative DNA-binding proteins, three are RNA-binding proteins, and four bind nucleotides.

### 6.5.6 Identification of Lipid-binding Proteins

The proteome chips of the invention are valuable for identifying activities that might not be accessible by other experimental approaches such as, for example, protein-drug interactions and protein-lipid interactions. Indeed, genome-wide analysis of proteins that bind to phospholipids has not been explored previously for any species. A proteome chip of the invention was used to study proteins that interact with phosphatidylinositol ("PI"). In addition to their roles as constituents of cellular membranes, phosphatidylinositols are important second-messengers that regulate diverse cellular processes, including growth, differentiation, cytoskeletal rearrangements, and membrane trafficking, and are found in the nucleus, vacuole, and plasma membrane (Odorizzi et al., 2000, TIBS 25:229; Fruman et al., 1998, Annu. Rev. Biochem. 67:481; Martin, 2000, Annu. Rev. Cell Dev. Bio. 14:231; Wera et al., 2001, FEMS Yeast Res. 1406:17). Because they are often present only transiently and in low abundance within cells, phosphatidylinositols have not been characterized extensively in yeast, and consequently, little is known about proteins that bind particular phospholipids (Odorizzi et al., 2000, TIBS 25:229; Fruman et al., 1998, Annu. Rev. Biochem. 67:229; Martin, 2000, Annu. Rev. Cell Dev. Bio. 14:231; Wera et al., 2001, FEMS Yeast Res. 1406:1).

To identify PI-binding proteins in yeast, liposomes were used as probes because the liposome provides the most relevant physiological condition to assay the interactors. Six types of liposomes were used. Each contains phosphatidylcholine ("PC") with 1% (w/w) N-(biotinoyl)-1,2-dihexadecanoyl-sn-glycero-3-phosphoethanolamine, triethylammonium salt (biotin DHPE). The biotinylated lipid serves as a label that can be detected by Cy3-streptavidin (21). In addition to PC, the five other liposomes contain either 5% (w/w) PI(3)P, PI(4)P, PI(3,4)P$_2$, PI(4,5)P$_2$, or PI(3,4,5)P$_3$ (FIG. 3A). Each of these phospholipids has been found in yeast except PI(3,4,5)P3 (Odorizzi et al., 2000, TIBS 25:229; Fruman et al., 1998, Annu. Rev. Biochem. 67:481; Martin, 2000, Annu. Rev. Cell Dev. Bio. 14:231; Wera et al., 2001, FEMS Yeast Res. 1406:1).

The protein-liposome interaction was assayed as follows. Appropriate amounts of each lipid in chloroform were mixed and dried under nitrogen. The lipid mixture was resuspended in TBS buffer by vortexing. The liposomes were created by sonication. Blocked proteome chips were washed three to five times in PBS buffer, and the extra liquid on the glass surface was removed by tapping the slides vertically on a KIMWIPE™. 200 $\mu$l of liposome solution was added to the proteome chip and immediately covered by a hydrophobic plastic cover slip (GRACE BIO-LABS™, USA). After trapped air bubbles were removed, the chip was incubated in a humidity chamber at RT for one hour. To remove the cover slip, the chip was immersed into large volume of PBS buffer (50 ml), so that the slip would float away. The chip was then moved to a second PBS bath (>50 ml) and washed 3 X 5 min with shaking at RT. After removing extra liquid on the surface of the chip, about 100 $\mu$l of Cy3 or Cy5 conjugated streptavidin (PIERCE™, USA; 1:2000 to 1:4000 dilution) was added to the surface and covered by a hydrophobic plastic cover slip (GRACE BIO-LABS™, USA). The chip was incubated for greater than 30 min in the dark at RT. The chip was then washed as described above. To completely remove the liquid on the chip, the chip was spun to dryness at 1500-2000 rpm for 5-10 min at RT.

The six liposomes identified a total of 150 different interactors that produced signals significantly higher than the background. An algorithm was devised to assist in the identification of positive signals (42). Fifty-two (35%) of the interactors (i.e., lipid-binding proteins) correspond to uncharacterized proteins thus ascribing the first biochemical activities to those proteins. These results also indicate that many previously uncharacterized proteins have potentially important biochemical activities.

Of the 52 uncharacterized proteins, thirteen (25%) are predicted to be associated with membranes (Gerstein, 1998, Proteins 33:518). Many others contain basic stretches, which can mediate electrostatic interactions with the negatively charged phosphatidylinositols.

Of the 98 previously described interactors, the subcellular localization of 81 of these has been investigated (Costanzo et al., 2001, Nucleic Acids Res. 29:75). Most are either membrane-associated proteins or are involved in lipid metabolism. More specifically, 45 proteins are membrane-associated and either have known or predicted membrane-spanning regions (Gerstein, 1998, Proteins 33:518). Among these identified interactors are integral membrane proteins, proteins with lipid modifications (e.g., the glycosylphosphatidylinositol (GPI) anchor proteins Tos6p and Sps2p (Costanzo et al., 2001, Nucleic Acids Res. 29:75), the prenylated proteins, Gpa2p and mating pheromone a-factor (Ansari et al., 1999, J. Bio. Chem. 274:30052), and peripherally-associated proteins (e.g., Kcc4p and Myo4p which are found at the bud neck and/or cell periphery (Bohl, 2000, EMBO J. 19:5514; Bertrand et al., 1998, Mol. Cell 2:437)). Eight others are involved in lipid metabolism (e.g., Bpl1p), inositol ring phosphorylation (e.g., Kcs1p), or predicted to be involved in membrane/lipid function (e.g., Ylr020cp which has homology to triacylglycerol lipase).

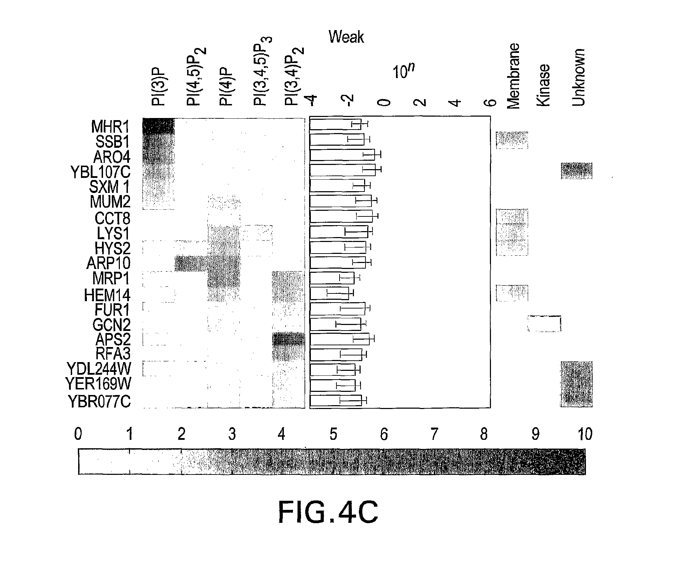

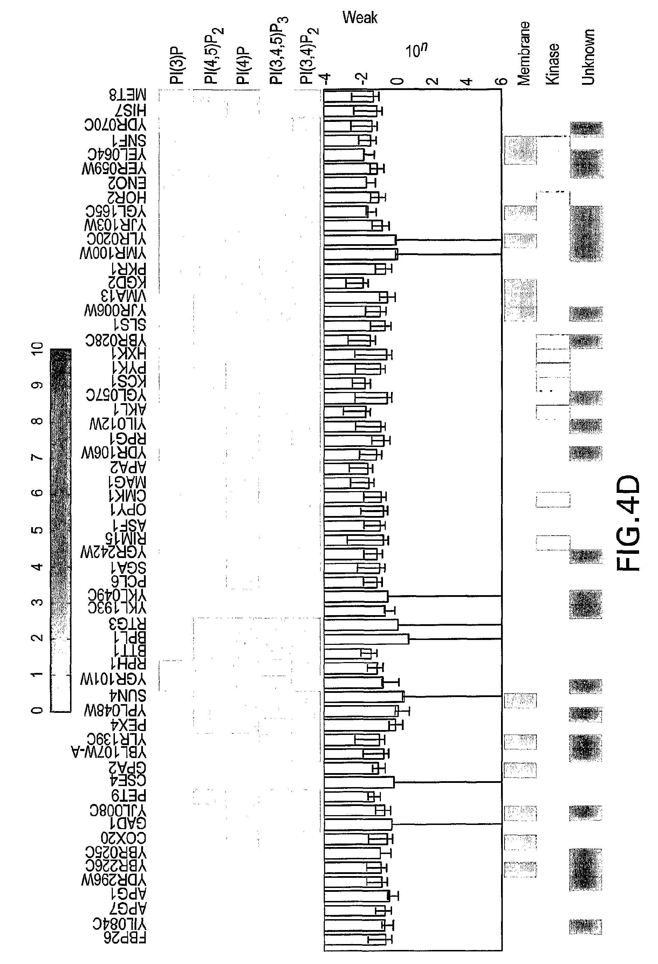

The interactors (i.e., phospholipid-binding proteins) were grouped in several ways (42). First, the interactors were sorted according to whether they bound lipids strongly or weakly, based on the phospholipid-binding signal relative to the amount of GST (FIG. 4). Slightly more (72%) of the strong lipid binding proteins (FIGS. 4A and 4B) were characterized relative to the weakly binding proteins (54%) (FIGS. 4C and 4D). Many of the strong lipid-binding proteins are membrane-associated, or are predicted to be associated with membranes, whereas fewer of the weaker binding proteins appear to be associated with membranes (FIG. 4, "Membrane" column). Surprisingly, nineteen of the lipid-binding proteins are kinases, and seventeen of these are protein kinases. Moreover, thirteen of the seventeen protein kinases bind very strongly to the phospholipids.

The proteins were also grouped by whether they preferentially bound one or more phosphatidylinositols as compared with phosphatidylcholine. One-hundred-and-one proteins bound to phosphatidylcholine as well or nearly as well as to the phosphatidylinositol lipids tested (PI/PC < 1.3) (FIGS. 4B and 4D). However, 49 proteins bound to one or more phosphatidylinositols preferentially (PI/PC > 1.3) (FIGS. 4A and 4C). Analysis of the strong interactors (i. e., phosphatidylinositol-binding proteins) revealed that many specifically bound particular phosphatidylinositol lipids. For example, Stp22p, which is required for vacuolar targeting of temperature-sensitive plasma membrane proteins, such as Ste2p and Can1p, preferentially binds PI(4)P (Li et al., 1999, Mol. Cell Biol. 19:3588). Nine protein kinases specifically bind PI(4)P and PI(3,4)P2 strongly and one binds these lipids weakly. Atp1p, the alpha subunit of F1-ATP synthase of the inner membrane of mitochondria, also preferentially binds PI(3,4)P2 (Arnold et al., 1999, J Biol. Chem). Sps2p, which is involved in middle/late stage of sporulation and is localized to the prospore membrane (Chu et al., 1998, Science 282: 699), preferentially interacts with PI(3)P. One interesting example is Myo4p which preferentially binds PI(4,5)P2; perhaps binding of this lipid is an important part of its interaction at the cell cortex and/or its regulation. No strong lipid binding targets were found that specifically bound PI(3,4,5)P3 although some proteins bound both this lipid and others (FIG. 4). These results demonstrate that many membrane-associated proteins including integral membrane proteins and peripherally associated proteins preferentially bind specific phospholipids in vivo.

Unexpectedly, many proteins involved in glucose metabolism, including regulators of glucose metabolism bind phospholipids. Specifically, three glucose metabolism enzymes, i.e., phosphoglycerate mutase (Gpm3p), enolase (Eno2p) and pyruvate kinase (Cdc19p/Pyk1p) which participate in sequential steps in glycolysis, were identified. Hexokinase (Hxk1p), which converts glucose to glucose-6-phosphate, and two protein kinases known to be regulators of glucose metabolism (i.e., Snflp and Rim15p) were also identified as lipid-binding interactors. Hxk1p has recently been shown to bind zwitterrion micelles which stimulate its activity (30); Eno2p has been shown to be secreted suggesting that it might also interact with membranes (31). Accordingly, in one embodiment, phospholipids can be used to regulate steps involved in glucose metabolism. In another embodiment, glycolytic steps can be enhanced by conducting steps of glucose metabolism on phospholipid scaffolds such as, but not limited to, cell membranes.

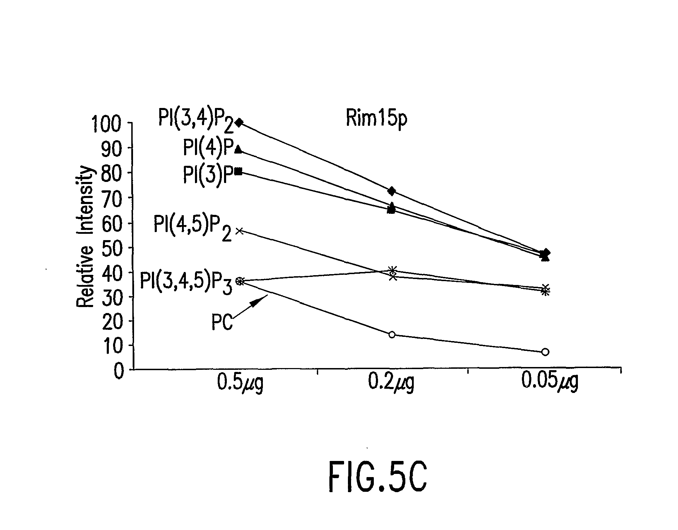

Surprisingly, the probes comprising phospholipids recognized many proteins not expected to be involved in membrane function or lipid signaling. Therefore, six proteins, *i.e.*, Rim15p, Eno2p, Hxk1p, Sps1p, Ygl059wp, and Gcn2p, were further tested for phosphoinositol binding using two types of standard assays (Williamson, 2000, Nat. Struct Biol. 7(10):834-7). For Rim15p, Eno2p, and Hxk1p, PI(4,5)P2 liposomes were first adhered to a nitrocellulose membrane, which was then blocked by BSA; different amounts of the GST fusion proteins and a GST control were used to probe the membrane, and bound proteins were detected using anti-GST antibodies. As shown in FIG. 5A, each yeast fusion protein tightly bound PI(4,5)P2 and exhibited a dosage effect; GST alone did not. We also carried out the reverse assay for four GST fusion proteins, Rim15p, Sps1p, Yg1059wp, and Gcn2p (Guerra et al., 2000, Biosci. Rep. 20: 41). Different amounts of these purified proteins were spotted onto nitrocellulose filters and probed with the six different liposomes (FIG. 5B). The bound liposomes were detected using a horseradish peroxidase ("HRP")-conjugated streptavidin. As with experiments using the proteome chips, liposomes bound to each protein, and did not bind the BSA control. Sps1p bound all five phosphoinositol- containing liposomes nearly equally. Rim15p Gcn2p, and Ygl059wp exhibited different affinities to different liposomes (*see* FIG. 5B for Rim15p). All three proteins bound strongest to PI(3)P and PI(4)P and PI(3,4)P2. For Rim15p, a linear correlation between the binding signal and the level of Rim15p was revealed (FIG. 5C).

Further, several proteins with unknown functions also showed strong lipid-binding activities, indicating that they are likely to bind phospholipids in cells. Routine assays known to those skilled in the art can be used to quickly screen lipid-binding interactors that are identified with a proteome chip to determine lipid-binding activity *in vivo.* Additionally, in accordance with the invention, a proteome chip can be used to determine if these interactors are involved in phospholipid metabolic pathways or signal transduction pathways.

In summary, these results unequivocally demonstrate that many lipid-binding proteins, including proteins not previously known to bind lipids, can be detected and characterized using a proteome chip of the invention. Moreover, phospholipid-binding interactors identified using the proteome chips of the invention can be shown to be *bona fide* lipid-binding proteins in conventional assays.

Moreover, because such interactions cannot be studied by using either the yeast two-hybrid system or DNA/oligo microarray technology, the proteome chips of the invention and the methods of using them represent a pioneering approach to exploring molecule-protein interactions.

These studies indicate that it is feasible to prepare and screen a proteome microarray for a eucaryote. The novel and unobvious combination of several technological features were critical for practicing the present invention. First, the proteins analyzed in this study were prepared from yeast expression clones that have been verified by DNA sequencing and contain pure plasmids. Second, it was essential to produce defined clones and proteins in a high throughput fashion. The procedures described herein provide enough protein for 5,000 protein chips. Third, the proteins were produced in a eucaryotic host. As such, large numbers of full-length proteins that are properly folded, posttranslationally modified and/or complexed with their native partners can be produced. As demonstrated by immunoblot analysis, at least 80% of the purified fusion proteins are expressed as full-length proteins.

Many interactors can bind to probes through associated proteins, and not directly. In some cases, the interactor can be part of a multimer. However, such associated proteins can also be detected and identified using the methods of the present invention. Nevertheless, the interactors detected by the methods of the invention likely directly interact with, or at least are tightly associated with a protein interacting with, a probe. Proteins of the proteome chips of the invention were prepared using stringent conditions (e.g., washed with 0.5 M NaCl), and Coomasie staining revealed only those bands detectable by anti-GST antibodies indicating that contamination with other proteins was minimal.

The collection of proteins assembled is likely to underrepresent secreted proteins with properly folded extracellular domains. A GST tag and a HisX6 tag was fused upstream of the translational initiation codon, such that membranous proteins having a signal peptide may not be delivered to the secretory pathway and may not be folded or modified appropriately. Three proteins having signal peptides were identified in the screens for lipid-binding proteins, suggesting that at least some secreted proteins are produced and contain functional domains.

Further, because not all proteins are readily overproduced and purified using the high-throughput methods practiced, not all interactions were detected. The protein arrays used are estimated to contain approximately 80% of the full-length yeast proteins at reasonable levels for screening, however, and thus most protein interactions can be expected to be detected.

These results demonstrate that a proteome chip can be screened for biochemical activities, thereby allowing global proteome analysis. Similar procedures can be used to prepare protein arrays of 10-100,000 proteins for global high-throughput proteome analysis in humans and other eukaryotes.

References and notes:

1 S. Fields, Y. Kohara, D. J. Lockhart, Proc. Natl. Acad. Sci. 96, 8825 (1999); A. Goffeau et al., Science 274, 563 (1996).
2 P. Ross-Macdonald et al., Nature 402, 413 (1999); J. L., DeRisi, V. R. Iyer, P.O. Brown, Science 278, 680 (1997); E. A. Winzeler et al., Science 285, 901 (1999). P. Uetz et al., Nature 403,623 (2000); T. Ito et al., Proc. Natl. Acad. Sci. USA. 97, 1143 (2000)
3 H. Zhu, M. Snyder, Curr. Opin. Chem. Biol. 5, 40 (2001).
4 M. R. Martzen et al., Science 286, 1153 (1999).
5 H. Zhu et al., Nat. Genet. 26, 283 (2000).
6 G. MacBeath, S. L. Schreiber, Science 289, 1760 (2000).
7 A. Caveman, J. Cell Sci. 113, 3543 (2000).
8 P. Arenkov et al., Anal. Biochem 278, 123 (2000).
9 D. A. Mitchell, T. K. Marshall, R. J. Deschenes, Yeast 9, 715 (1993). The expression vector pEGH was created by inserting an RGS-HisX6 epitope tag between the GST gene and the polycloning site of pEG(KG). The yeast ORFs were cloned using the strategy described previously (5), except every step was done in a 96-well format. Plasmid DNAs confirmed by DNA sequencing were reintroduced into both yeast (Y258) and *E. coli* (DH5a). The library contains 5800 unique ORFs.
10 Details of a 96-well format protein purification protocol, a full list of results from all the experiments, and the design of the positive identification algorithms, can be found at public web site bioinfo.mbb.yale.edu/proteinchip.
11 Biotinylated calmodulin (CalBiochem, USA) was added to the proteome chip at 0.02 mg/ml in PBS buffer with 0.1 mM calcium and incubated in a humidity chamber for one hour at room temperature. 0.1 mM calcium was present in buffers in all subsequent steps. The chip was washed three times with PBS at RT. Cy3-conjugated streptavidin (Jackson IR, USA) (1:5000 dilution) was added to the chip and incubated for 30 min at RT. After extensive washing, the chip was spun dry and scanned using a microarray scanner; the data was subsequently acquired with the GENEPIX™ array densitometry software (Axon, USA).
12 S. S. Hook, A. R. Means, Annu. Rev. Pharmacol. Toxicol.41, 471 (2001).
13 M. S. Cyert, R. Kunisawa, D. Kaim, J. Thorner, Proc. Natl. Acad. Sci. USA 88, 7376 (1991).
14 D. A. Stirling, K. A. Welch, M. J. Stark, EMBO J. 13,4329 (1994).
15 F. Bohl, Kruse, EMBO J. 19, 5514 (2000); E. Bertrand et al., Mol. Cell 2, 437 (1998).
16 D. C. Winter, E. Y. Choe, R. Li, Proc. Nat. Acad. Sci. USA 96, 7288 (1999).
17 C. Schaerer-Brodbeck, H. Riezman, Mol. Biol. Cell 11, 1113 (2000).
18 K. Homma, J. Saito, R. Ikebe, M. Ikebe, J. Biol. Chem. 275, 34766 (2000).
19 J. Menendez, J. Delgado, C. Gancedo, Yeast 14, 647 (1998).
20 G. Odorizzi, M. Babst, S. D. Emr, TIBS 25,229 (2000); D. A. Fruman et al., Annu. Rev. Biochem. 67, 481 (1998); T. F. Martin, Annu. Rev. Cell Dev. Biol. 14, 231 (2000); S. Wera, J. C. T. Bergsma, FEMS Yeast Res. 1406, 1 (2001).
21 Liposomes were prepared using standard methods (52). Briefly, appropriate amounts of each lipid in chloroform were mixed and dried under nitrogen. The lipid mixture was resuspended in TBS buffer by vortexing. The liposomes were created by sonication. To probe the proteome chips, 60 ml of the different liposomes were added onto different chips. The chips were incubated in a humidity chamber for one hour at RT. After washing with TBS buffer for three times, Cy3-conjugated streptavidin (1:5000 dilution) was added to the chip and incubated for 30 min at RT.
22 Positives were identified using a combination of the GenePix software which computes a local intensity background for each spot and a series of algorithms we developed. Details can be found at: public web site bioinfo.mbb.yale.edu/proteinchip.
23 M. C. Costanzo et al., Nucleic Acids Res. 29, 75 (2001).
24 M. Gerstein, Proteins 33, 518 (1998).
25 K. Ansari et al., J. Biol. Chem. 274, 30052 (1999).
26 Y. Barral, M. Parra, S. Bidlingmaier, M. Snyder, Genes Dev. 13, 176 (1999).
27 Y. Li, T. Kane, C. Tipper, P. Spatrick, D. D. Jenness, Mol. Cell Biol. 19, 3588 (1999).
28 I. Arnold et al., JBiol. Chem. 274,36 (1999).
29 S. Chu et al., Science 282, 699 (1998).
30 A. Casamayor et al., Curr. Biol. 9, 186 (1999); R. Guerra, M. L. Bianconi, Biosci. Rep. 20, 41 (2000).
31 M. Pardo et al., Yeast 15,459 (1999).

**Claims**

1.  A positionally addressable array comprising a plurality of purified proteins, with each protein being at a different position on a solid support, wherein the plurality of purified proteins comprises at least 40% of all proteins expressed in a single species and wherein the species is a Eukaryote.

**2.** An array of claim 1, wherein the proteins are organized on the array according to a classification of proteins.

**3.** An array of claim 2, wherein the classification is by abundance, function, enzymatic activity, homology, protein family, association with a particular metabolic pathway, or posttranslational modification.

**4.** An array of any one of claims 1 to 3, wherein the proteins are attached to the solid support via a poly histidine (His) tag.

**5.** An array of any one of claims 1 to 4, wherein the solid support comprises nickel or a nickel-coated slide.

**6.** An array of any one of claims 1 to 5, wherein the solid support comprises a nitrocellulose-coated slide.

**7.** A method for detecting a binding protein comprising the steps of:

(a) contacting a probe with a positionally addressable array of claim 1 and
(b) detecting any protein-probe interaction.

**8.** An array of claim 1, wherein the proteins are attached to the solid support via a biotin tag.

**9.** The array of claim 1, wherein the solid support comprises a nitrocellulose coated slide.

**10.** The array of claim 1, wherein the proteins are mammalian.

**11.** The array of claim 10, wherein the proteins are human.

**12.** The array of claim 1, wherein the proteins arc fusion proteins.

**13.** The array of claim 12, wherein the proteins comprise a first tag and a second tag.

**14.** The array of claim 13, wherein the first tag is a glutathione S transferase (GST) tag and the second tag is poly histidine (His) tag.

**15.** The array of claim 1, wherein the plurality of proteins comprises substrates for different classes of enzymes.

**16.** The array of claim 15, wherein the plurality of proteins comprises substrates for protein kinases, phosphatases, proteases, glycosidases and acetylases.

**17.** The array of claim 1, wherein the plurality of proteins comprises proteins that bind kinases, proteases, hormones, DNA, RNA, phosphatases, glycosidases and acetylases.

**Patentansprüche**

**1.** Positionsmäßig adressierbare Anordnung aufweisend eine Mehrzahl von gereinigten Proteinen, wobei sich jedes Protein an einer unterschiedlichen Position auf einem festen Träger befindet, wobei die Mehrzahl von gereinigten Proteinen mindestens 40% aller in einer einzigen Spezies exprimierten Proteine aufweist, und wobei die Spezies ein Eukaryot ist.

**2.** Anordnung nach Anspruch 1, wobei die Proteine auf der Anordnung entsprechend einer Protein-Klassifizierung organisiert sind.

**3.** Anordnung nach Anspruch 2, wobei die Klassifizierung nach Häufigkeit, Funktion, enzymatischer Aktivität, Homologie, Proteinfamilie, Verbindung mit einem bestimmten Stoffwechselweg oder posttranslationaler Modifikation erfolgt.

**4.** Anordnung nach einem der Ansprüche 1 bis 3, wobei die Proteine mittels eines Polyhistidin (His)-Anhangs an den festen Träger gebunden sind.

**5.** Anordnung nach einem der Ansprüche 1 bis 4, wobei der feste Träger Nickel oder einen Nickel-beschichteten Objektträger aufweist.

**6.** Anordnung nach einem der Ansprüche 1 bis 5, wobei der feste Träger einen Nitrocellulose-beschichteten Objektträger aufweist.

**7.** Verfahren zum Nachweisen eines Bindungsproteins, folgende Schritte aufweisend:

(a) in Kontakt Bringen einer Sonde mit einer positionsmäßig adressierbaren Anordnung nach Anspruch 1 und
(b) Nachweisen irgendeiner Protein-Sonden-Wechselwirkung.

**8.** Anordnung nach Anspruch 1, wobei die Proteine mittels eines Biotin-Anhangs an den festen Träger gebunden sind.

**9.** Anordnung nach Anspruch 1, wobei der feste Träger einen Nitrocellulosebeschichteten Objektträger aufweist.

**10.** Anordnung nach Anspruch 1, wobei die Proteine von Säugetieren sind.

**11.** Anordnung nach Anspruch 10, wobei die Proteine menschlich sind.

**12.** Anordnung nach Anspruch 1, wobei die Proteine Fusionsproteine sind.

**13.** Anordnung nach Anspruch 12, wobei die Proteine einen ersten Anhang und einen zweiten Anhang aufweisen.

**14.** Anordnung nach Anspruch 13, wobei der erste Anhang ein Glutathion S-Transferase (GST)-Anhang ist und der zweite Anhang ein Polyhistidin (His)-Anhang ist.

**15.** Anordnung nach Anspruch 1, wobei die Mehrzahl von Proteinen Substrate für unterschiedliche Klassen von Enzymen aufweist.

**16.** Anordnung nach Anspruch 15, wobei die Mehrzahl von Proteinen Substrate für Protein-Kinasen, -Phosphatasen, -Proteasen, -Glycosidasen und - Acetylasen aufweist.

**17.** Anordnung nach Anspruch 1, wobei die Mehrzahl von Proteinen Proteine aufweist, die Kinasen, Proteasen, Hormone, DNA, RNA, Phosphatasen, Glycosidasen und Acetylasen binden.

**Revendications**

**1.** Puce adressable en position comprenant une pluralité de protéines purifiées, chaque protéine étant à une position différente sur un support solide, dans laquelle la pluralité de protéines purifiées comprend au moins 40 % de l'ensemble des protéines exprimées dans une espèce unique et l'espèce étant un eucaryote.

**2.** Puce de la revendication 1, dans laquelle les protéines sont organisées sur la puce selon une classification de protéines.

**3.** Puce de la revendication 2, dans laquelle la classification est effectuée par abondance, fonction, activité enzymatique, homologie, famille de protéines, association avec une voie métabolique particulière, ou modification post-traductionnelle.

**4.** Puce de l'une quelconque des revendications 1 à 3, dans laquelle les protéines sont liées au support solide par l'intermédiaire d'une étiquette polyhistidine (His).

**5.** Puce de l'une quelconque des revendications 1 à 4, dans laquelle le support solide comprend du nickel ou une lame revêtue de nickel.

**6.** Puce de l'une quelconque des revendications 1 à 5, dans laquelle le support solide comprend une lame à base de nitrocellulose.

**7.** Procédé de détection d'une protéine de liaison comprenant les étapes de :

(a) mise en contact d'une sonde avec une puce adressable en position de la revendication 1 et

(b) détection d'une interaction protéine-sonde.

**8.** Puce de la revendication 1, dans lequel les protéines sont liées au support solide par l'intermédiaire d'une étiquette de biotine.

**9.** Puce de la revendication 1, dans laquelle le support solide comprend une lame revêtue de nitrocellulose.

**10.** Puce de la revendication 1, dans laquelle les protéines sont de mammifère.

**11.** Puce de la revendication 10, dans laquelle les protéines sont humaines.

**12.** Puce de la revendication 1, dans laquelle les protéines sont des protéines de fusion.

**13.** Puce de la revendication 12, dans laquelle les protéines comprennent une première étiquette et une deuxième étiquette.

**14.** Puce de la revendication 13, dans laquelle la première étiquette est une étiquette de glutathion S-transférase (GST) et la deuxième étiquette est une étiquette de polyhistidine (His).

**15.** Puce de la revendication 1, dans laquelle la pluralité de protéines comprend des substrats pour différentes classes d'enzymes.

**16.** Puce de la revendication 15, dans laquelle la pluralité de protéines comprend des substrats pour des protéine kinases, des phosphatases, des protéases, des glycosidases et des acétylases.

**17.** Puce de la revendication 1, dans laquelle la pluralité de protéines comprend des protéines qui se lient à des kinases, des protéases, des hormones, un ADN, un ARN, des phosphatases, des glycosidases et des acétylases.

FIG. 1A

FIG.1B

To
FIG.2B

To
FIG.2B

FIG.2A

FIG.2B

FIG.2C

FIG.3A

| | | |
|---|---|---|
| YFL003C/MSH4 | LKET LQS VKSLKD AL | 390 |
| YJR073C/OPI3 | HSVL LQS SKFQLA IV | 28 |
| YBR050C/REG2 | DEHF IQR LPSTRL NS | 196 |
| YNL202W/SPS19 | AKIF LQR LGSTRD IA | 246 |
| YOL016C/CMK2 | DDLF LQS QKKGGE LT | 395 |
| YBR011C/IPP1 | LNPI IQD TKKGKL RF | 80 |
| YGR034W/RPL26B | RKAI IQR .KGGKL E. | 129 |
| YFR004W/RPN11 | VDPF IQS VKGKVV ID | 154 |
| YIL021W/RPB3 | GHPI IQD .KEGNG VL | 143 |
| YGL063W/PUS2 | RVWG IQP VNKKFN AR | 103 |
| YDR292C/SRP101 | LLRF IQS .KRSKD EE | 388 |
| YFR014C/CMK1 | LNMF IQK LRDLYL EQ | 346 |
| YBR213W/MET8 | DLFG IQH CHN.ID VK | 242 |
| YAL029C/MYO4 | NGLI IQS SKFISK VL | 1167 |

FIG.3B

FIG.4A

FIG.4B

FIG.4C

FIG.4D

EP 1 392 342 B1

FIG.5A

FIG.5B

FIG.5C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 60290583 B
- US 60308149 B
- WO 0183827 A
- US 09849781 B
- US 8399383 B
- WO 9859361 A

**Non-patent literature cited in the description**

- **FIELDS et al.** *Proc Natl Acad Sci.,* 1999, vol. 96, 8825
- **GOFFEAU et al.** *Science,* 1996, vol. 274, 563
- **ROSS-MACDONALD et al.** *Nature,* 1999, vol. 402, 413
- **DERISI et al.** *Science,* 1997, vol. 278, 680
- **WINZELER et al.** *Science,* 1999, vol. 285, 901
- **UETZ et al.** *Nature,* 2000, vol. 403, 623
- **ITO et al.** *Proc. Natl. Acad. Sci. U. S. A.,* 2000, vol. 97, 1143
- **ZHU et al.** *Curr. Opin. Chem. Biol.,* 2001, vol. 5, 40
- **MARTZEN et al.** *Science,* 1999, vol. 286, 1153
- **ZHU et al.** *Nat. Genet.,* 2000, vol. 26, 283
- **MACBEATH.** *Science,* 2000, vol. 289, 1760
- **CAVEMAN.** *J. Cell Sci.,* 2000, vol. 113, 3543
- **MACBEATH et al.** *Science,* 2000, vol. 289, 1763
- **ARENKOV et al.** *Anal. Biochem,* 2000, vol. 278, 123
- **ZHU, HENG et al.** *Curr. Opin. Chem. Biol.,* 2001, vol. 5 (1), 40-45
- **KUMAR, ANUJ et al.** *Nat. Rev. Gen,* 2001, vol. 2 (4), 302-312
- **BUSSOW et al.** *Genomics,* 2000, vol. 65, 1
- **CASAMAYOR et al.** *Curr. Biol.,* 1999, vol. 9, 186
- **GUERRA et al.** *Biosci. Rep.,* 2000, vol. 20, 41
- **G. KOVACS.** Micromachined Transducers Sourcebook. Academic Press, 1998
- **M. MADOU.** Fundamentals of Microfabrication. CRC Press, 1997
- **HATCH et al.** Rolling circle amplification of DNA immobilized on solid surfaces and its application to multiplex mutation detection. *Genet. Anal,* 1999, vol. 15, 35-40
- **FINI et al.** Development of a chemiluminescence competitive PCR for the detection and quantification of parvovirus B 19 DNA using a microplate luminometer. *Clin Chem.,* 1999, vol. 45, 1391-6
- **KRUSE et al.** Detection and quantitative measurement of transforming growth factor-betal (TGF-betal) gene expression using a semi-nested competitive PCR assay. *Cytokine,* 1999, vol. 11, 179-85
- **GUENTHNER ; HART.** Quantitative, competitive PCR assay for HIV-1 using a microplate-based detection system. *Biotechniques,* 1998, vol. 24, 810-6
- **LAKEY et al.** Measuring protein-protein interactions. *Curr Opin Struct Biol.,* 1998, vol. 8, 119-23
- **PALLADINO et al.** *Cancer Res.,* 1987, vol. 47, 5074-9
- **BLACHERE et al.** *J. Immunotherapy,* 1993, vol. 14, 352-6
- **AMES et al.** *J. Immunol. Methods,* 1995, vol. 184, 177-86
- **KETTLEBOROUGH et al.** *Eur. J. Immunol.,* 1994, vol. 24, 952-8
- **PERSIC et al.** *Gene,* 1997, vol. 187, 9-18
- **MITCHELL et al.** *Yeast,* 1993, vol. 9, 715
- **ZHU et al.** *Nat Genet.,* 2000, vol. 26, 283
- **MACBEATH et al.** *Science,* 2000, vol. 289, 1760
- **HOOK et al.** *Annu. Rev. Pharmacol. Toxicol.,* 2001, vol. 41, 471
- **HOOK et al.** *Annu. Rev. Pharmacol. Toxicol.,* 2001, vol. 41
- **CYERT et al.** *Proc. Natl. Acad. Sci. U.S.A.,* 1991, vol. 88, 7376
- **STIRLING et al.** *EMBO J.,* 1994, vol. 13, 4329
- **BOHL et al.** *EMBO J.,* 2000, vol. 19, 5514
- **BERTRAND et al.** *Mol. Cell,* 1998, vol. 2, 437
- **WINTER et al.** *Proc. Nat. Acad. Sci. U.S.A.,* 1999, vol. 96, 7288
- **SCHAERER-BRODBECK et al.** *Mol. Biol. Cell,* 2000, vol. 11, 1113
- **MENENDEZ et al.** *Yeast,* 1998, vol. 14, 647

- **HOMMA et al.** *J. Biol. Chem,* 2000, 275
- **WILLIAMSON.** *Nat. Struct. Biol.,* 2000, vol. 7 (10), 834-7
- **COSTANZO et al.** *Nucleic Acids Res.,* 2001, vol. 29, 75
- **STERNBERG. et al.** *Cell,* 1987, vol. 48, 567
- **STERNBERG et al.** *Cell,* 1987, vol. 48, 567
- **THOMAS et al.** *Mol. Cell Biol.,* 1995, vol. 15, 6526
- **SADEKOVA et al.** *Curr. Genet.,* 1996, vol. 30 (1), 50-5
- **LANKER et al.** *Cell,* 1992, vol. 70, 647
- **SUZUKI et al.** *Curr. Genet,* 1990, vol. 17, 185
- **COSTANZO.** *Nucleic Acids Res.,* 2001, vol. 29, 75
- **FROMONTRACINE et al.** *Yeast,* 2000, vol. 17, 95
- **SCHWIKOWSKI et al.** *Nat. Biot.,* 2000, vol. 18, 1257
- **ODORIZZI et al.** *TIBS,* 2000, vol. 25, 229
- **FRUMAN et al.** *Annu. Rev. Biochem.,* 1998, vol. 67, 481
- **MARTIN.** *Annu. Rev. Cell Dev. Bio.,* 2000, vol. 14, 231
- **WERA et al.** *FEMS Yeast Res.,* 2001, vol. 1406, 17
- **FRUMAN et al.** *Annu. Rev. Biochem.,* 1998, vol. 67, 229
- **WERA et al.** *FEMS Yeast Res.,* 2001, vol. 1406, 1
- **GERSTEIN.** *Proteins,* 1998, vol. 33, 518
- **ANSARI et al.** *J. Bio. Chem.,* 1999, vol. 274, 30052
- **BOHL.** *EMBO J.,* 2000, vol. 19, 5514
- **LI et al.** *Mol. Cell Biol.,* 1999, vol. 19, 3588
- **ARNOLD et al.** *J Biol. Chem,* 1999
- **CHU et al.** *Science,* 1998, vol. 282, 699
- **WILLIAMSON.** *Nat. Struct Biol,* 2000, vol. 7 (10), 834-7
- **S. FIELDS ; Y. KOHARA ; D. J. LOCKHART.** *Proc. Natl. Acad. Sci.,* 1999, vol. 96, 8825
- **A. GOFFEAU et al.** *Science,* 1996, vol. 274, 563
- **P. ROSS-MACDONALD et al.** *Nature,* 1999, vol. 402, 413
- **J. L., DERISI ; V. R. IYER ; P.O. BROWN.** *Science,* 1997, vol. 278, 680
- **E. A. WINZELER et al.** *Science,* 1999, vol. 285, 901
- **P. UETZ et al.** *Nature,* 2000, vol. 403, 623
- **T. ITO et al.** *Proc. Natl. Acad. Sci. USA.,* 2000, vol. 97, 1143
- **H. ZHU ; M. SNYDER.** *Curr. Opin. Chem. Biol.,* 2001, vol. 5, 40
- **M. R. MARTZEN et al.** *Science,* vol. 286, 1153
- **H. ZHU et al.** *Nat. Genet.,* 2000, vol. 26, 283
- **G. MACBEATH ; S. L. SCHREIBER.** *Science,* 2000, vol. 289, 1760
- **A. CAVEMAN.** *J. Cell Sci.,* 2000, vol. 113, 3543
- **P. ARENKOV et al.** *Anal. Biochem,* 2000, vol. 278, 123
- **D. A. MITCHELL ; T. K. MARSHALL ; R. J. DE-SCHENES.** *Yeast,* 1993, vol. 9, 715
- **S. S. HOOK ; A. R. MEANS.** *Annu. Rev. Pharmacol. Toxicol.,* 2001, vol. 41, 471
- **M. S. CYERT ; R. KUNISAWA ; D. KAIM ; J. THORNER.** *Proc. Natl. Acad. Sci. USA,* 1991, vol. 88, 7376
- **D. A. STIRLING ; K. A. WELCH ; M. J. STARK.** *EMBO J.,* 1994, vol. 13, 4329
- **F. BOHL, KRUSE.** *EMBO J.,* 2000, vol. 19, 5514
- **E. BERTRAND et al.** *Mol. Cell,* 1998, vol. 2, 437
- **D. C. WINTER ; E. Y. CHOE ; R. LI.** *Proc. Nat. Acad. Sci. USA,* 1999, vol. 96, 7288
- **C. SCHAERER-BRODBECK ; H. RIEZMAN.** *Mol. Biol. Cell,* 2000, vol. 11, 1113
- **K. HOMMA ; J. SAITO ; R. IKEBE ; M. IKEBE.** *J. Biol. Chem.,* 2000, vol. 275, 34766
- **J. MENENDEZ ; J. DELGADO ; C. GANCEDO.** *Yeast,* 1998, vol. 14, 647
- **G. ODORIZZI ; M. BABST ; S. D. EMR.** *TIBS,* 2000, vol. 25, 229
- **D. A. FRUMAN et al.** *Annu. Rev. Biochem.,* 1998, vol. 67, 481
- **T. F. MARTIN.** *Annu. Rev. Cell Dev. Biol.,* 2000, vol. 14, 231
- **S. WERA ; J. C. T. BERGSMA.** *FEMS Yeast Res.,* 2001, vol. 1406, 1
- **M. C. COSTANZO et al.** *Nucleic Acids Res.,* 2001, vol. 29, 75
- **M. GERSTEIN.** *Proteins,* 1998, vol. 33, 518
- **K. ANSARI et al.** *J. Biol. Chem.,* 1999, vol. 274, 30052
- **Y. BARRAL ; M. PARRA ; S. BIDLINGMAIER ; M. SNYDER.** *Genes Dev.,* 1999, vol. 13, 176
- **Y. LI ; T. KANE ; C. TIPPER ; P. SPATRICK ; D. D. JENNESS.** *Mol. Cell Biol.,* 1999, vol. 19, 3588
- **I. ARNOLD et al.** *JBiol. Chem.,* 1999, vol. 274, 36
- **S. CHU et al.** *Science,* 1998, vol. 282, 699
- **A. CASAMAYOR et al.** *Curr. Biol.,* 1999, vol. 9, 186
- **R. GUERRA ; M. L. BIANCONI.** *Biosci. Rep.,* 2000, vol. 20, 41
- **M. PARDO et al.** *Yeast,* 1999, vol. 15, 459

SEQUENCE LISTING

<110> Yale University

<120> GhOBAT, ANALYSIS OF PROTEIN ACTIVITIES
USING PROTEOME CHIPS

<130> 6523-035-228

<140> To be assigned
<141> Herewith

<150> 60/290,583 <151> 2001-05-11

<150> 60/308,149 <151> 2001-07-26

<160> 16

<170> FastSEQ for Windows Version 4.0

<210> 1
<211> 7
<212> PRT
<213> Artificial Sequence

<220>
<223> consensus sequence for calmodulin-binding protein

<221> VARIANT
<222> 1
<223> Xaa = Ile or Leu

<221> VARIANT
<222> 3
<223> Xaa = any amino acid

<221> VARIANT
<222> 5
<223> Xaa = Lys or any amino acid

<221> VARIANT
<222> 7
<223> Xaa = basic amino acid

<400> 1

```
Xaa Gln Xaa Lys Xaa Gly Xaa
 1               5
```

<210> 2
<211> 17
<212> PRT
<213> Saccharomyces cerevisiae

<400> 2

```
Leu Lys Glu Thr Leu Gln Ser Val Lys Ser Leu Lys Asp Ala Leu Asn
 1               5                   10                  15
Asp
```

<210> 3
<211> 17
<212> PRT
<213> Saccharomyces cerevisiae

<400> 3

```
His Ser Val Asp Leu Gln Ser Ser Lys Phe Gln Leu Ala Ile Val Cys
 1               5                   10                  15
Thr
```

<210> 4
<211> 17
<212> PRT
<213> Saccharomyces cerevisiae

<400> 4

```
Asp Glu His Phe Ile Gln Arg Leu Pro Ser Thr Arg Leu Asn Ser Thr
 1               5                   10                  15
Asp
```

<210> 5
<211> 17
<212> PRT
<213> Saccharomyces cerevisiae

<400> 5

```
Ala Lys Ile Pro Leu Gln Arg Leu Gly Ser Thr Arg Asp Ile Ala Glu
 1               5                   10                  15
Ser
```

<210> 6
<211> 17
<212> PRT
<213> Saccharomyces cerevisiae

<400> 6

```
Asp Asp Leu Arg Leu Gln Ser Gln Lys Lys Gly Gly Glu Leu Thr Glu
 1               5                   10                  15
Glu
```

<210> 7
<211> 17
<212> PRT
<213> Saccharomyces cerevisiae

<400> 7

Leu Asn Pro Ile Ile Gln Asp Thr Lys Lys Gly Lys Leu Arg Phe Val
1               5               10              15
Arg

<210> 8
<211> 13
<212> PRT
<213> Saccharomyces cerevisiae

<400> 8

Arg Lys Ala Leu Ile Gln Arg Lys Gly Gly Lys Leu Glu
1               5               10

<210> 9
<211> 17
<212> PRT
<213> Saccharomyces cerevisiae

<400> 9

Val Val Asp Pro Ile Gln Ser Val Lys Gly Lys Val Val Ile Asp Ala
1               5               10              15
Phe

<210> 10
<211> 16
<212> PRT
<213> Saccharomyces cerevisiae

<400> 10

Gly His Pro Ile Ile Gln Asp Lys Glu Gly Asn Gly Val Leu Ile Cys
1               5               10              15

<210> 11
<211> 17
<212> PRT
<213> Saccharomyces cerevisiae

<400> 11

Arg Val Trp Gly Ile Gln Pro Val Asn Lys Lys Phe Asn Ala Arg Ser
1               5               10              15
Ala

<210> 12
<211> 17
<212> PRT
<213> Saccharomyces cerevisiae

<400> 12

Leu Leu Arg Glu Ile Gln Ser Lys Arg Ser Lys Lys Asp Glu Glu Gly
1               5               10              15
Lys

<210> 13
<211> 17
<212> PRT
<213> Saccharomyces cerevisiae

<400> 13

```
Leu Asn Met Lys Ile Gln Lys Leu Arg Asp Leu Tyr Leu Glu Gln Thr
 1               5               10                  15
Glu
```

<210> 14
<211> 16
<212> PRT
<213> Saccharomyces cerevisiae

<400> 14

```
Asp Leu Phe Gly Ile Gln His Cys His Asn Ile Asp Val Lys Arg Leu
 1               5               10                  15
```

<210> 15
<211> 17
<212> PRT
<213> Saccharomyces cerevisiae

<400> 15

```
Asn Gly Leu Leu Ile Gln Ser Ser Lys Phe Ile Ser Lys Val Leu Leu
 1               5               10                  15
Thr
```

<210> 16
<211> 11
<212> PRT
<213> Artificial Sequence

<220>
<223> motif found in many calmodulin-binding proteins

<221> VARIANT
<222> 3, 4, 5, 6, 8, 9, 10
<223> Xaa = Any Amino Acid

<400> 16

```
Ile Gln Xaa Xaa Xaa Xaa Lys Xaa Xaa Xaa Arg
 1               5               10
```